# EUROPÄISCHE PATENTSCHRIFT

(11) **EP 0 389 477 B1**
(45) Veröffentlichungstag und Bekanntmachung des Hinweises auf die Patenterteilung: **12.05.1993**
(21) Anmeldenummer: 88907200.5
(22) Anmeldetag: 29.08.1988
(51) Int. Cl.: A61M 5/24

(54) **INJEKTIONSVORRICHTUNG MIT EINER VERFORMBAREN AMPULLE**
INJECTION DEVICE FOR USE WITH A DEFORMABLE AMPOULE
DISPOSITIF D'INJECTION UTILISE AVEC UNE AMPOULE DEFORMABLE

(30) Priorität: 09.09.1987 AT 2289/87
(43) Veröffentlichungstag der Anmeldung: 03.10.1990
(73) Patentinhaber: PICKHARD, Ewald, 1160 Wien (AT)
(72) Erfinder: PICKHARD, Ewald, 1160 Wien (AT)
(74) Vertreter: Secklehner, Günter, Dr.
(86) Internationale Anmeldenummer: AT8800068
(87) Internationale Veröffentlichungsnummer: WO8902286

(56) Entgegenhaltungen:
- EP-A- 0 162 791
- EP-A- 0 170 784
- CH-A- 468 196
- DE-A- 2 112 654
- DE-C- 823 177
- GB-A- 2 072 017
- US-A- 3 372 697
- US-A- 3 605 744
- US-A- 3 884 229
- US-A- 4 150 672

## Beschreibung

Die Erfindung betrifft eine Injektionsvorrichtung, wie sie im Oberbegriff des Patentanspruches 1 definiert und aus der EP-A-0 170 784 bekannt ist.

Es sind bereits verschiedene Injektionsvorrichtungen bekannt, in welchen eine verformbare Ampulle verwendet werden kann. Eine derartige Injektionsvorrichtung - gemäß EP-A1-0 170 784 weist ein aufklappbares Gehäuse auf, in dem eine Aufnahme für eine Faltenbalgampulle vorgesehen ist. Die Faltenbalgampulle wird im Bereich eines ihrer Stirnenden direkt im Gehäuse positioniert und dem gegenüberliegenden Stirnende der Faltenbalgampulle ist eine Antriebsvorrichtung zugeordnet. Diese besteht aus einem Stempel, der über eine Spindel-Wandermutteranordnung in Längsrichtung der Faltenbalgampulle in Richtung des Auslasses derselben verstellbar ist. Die Gewindespindel wird von einem elektrischen Antriebsmotor in Rotation versetzt. Dabei ist eine Verbindungsstange des Kolbens mit der Spindel als Führungsteil ausgebildet und gleitet entlang eines in einer Wand des Gehäuses ausgebildeten Schlitzes, welcher die Führungsbahn darstellt. In das aus dem Gehäuse vorragende Ende der Faltenbalgampulle kann eine Spritzennadel eingesetzt werden. Je nach der Vorschubgeschwindigkeit des Kolbens wird das in der Faltenbalgampulle enthaltene Medikament über einen kürzeren oder längeren Zeitraum verteilt durch die Spritzennadel hinausgepreßt. Bei dieser Injektionsvorrichtung ist die Handhabung der Faltenbalgampulle beim Einlegen in das Gehäuse schwierig. Darüberhinaus ist die Dosierung des Medikamentes nicht exakt möglich.

Bei einer weiteren bekannten Injektionsvorrichtung - gemäß CH-A 468 196 - ist eine elastisch verformbare Ampulle vorgesehen, die in einer rohrförmigen Stützvorrichtung verschiebbar angeordnet ist und mit ihrer vorderen Stirnwand an einem Kolben befestigt ist, der in der Stützvorrichtung mit leichtem Spiel axial geführt ist. Die Spritzennadel ist in einem zwischen der Stirnseite der Stützvorrichtung und der elastisch verformbaren Balgampulle angeordneten Zwischenteil relativ verschiebbar gehaltert. Zum Auspressen des Medikamentes aus der elastisch verformbaren Balgampulle muß von der der Injektionsnadel entgegengesetzten Seite in die Stützvorrichtung eine Kolbenstange eingeführt werden. Die Dosierungsmenge des Medikamentes kann nur durch das Gefühl der das Medikament verabreichenden Person gesteuert werden.

Eine andere Ausbildung einer Injektionsvorrichtung ist in der EP-A2-0 150 681 beschrieben. Diese Injektionsvorrichtung weist einen Gehäuseteil mit einem feststehenden Kolben auf, in welchem ein Kanal angeordnet ist, der erst dann eine Medikamentenabgabe ermöglicht, wenn die Aufnahmevorrichtung für das Medikament um eine vorbestimmte Distanz in Richtung der Spritzennadel bewegt worden ist. Weiters wird durch die Anordnung des Kanals ein dichtender Abschluß des Kolbens gegenüber der Innenwand der Aufnahmevorrichtung im sogenannten Lieferzustand vor der Benützung der Injektionsvorrichtung erreicht, sodaß kein Medikament ungewollt austreten kann.

Bei einer anderen bekannten Injektionsvorrichtung - gemäß US-A 4,525,164 - ist ebenfalls eine verformbare Ampulle vorgesehen. Diese, etwa in einem Halbkreis angeordnete, in Art eines Schlauches ausgebildete Ampulle ist im Bereich eines ihrer beiden Stirnenden mit einer Spritzennadel verbunden. Dem anderen Ende der Ampulle ist zu Beginn der Ausgabe des in der Ampulle enthaltenen Medikamentes eine Quetschrolle zugeordnet. Zur Ausgabe des Medikamentes aus der Ampulle wird die Quetschrolle in Richtung des mit der Spritzennadel verbundenen Stirnendes der Ampulle bewegt. Dabei wird die Ampulle zwischen der Quetschrolle und der dieser gegenüberliegenden Wand des die Ampulle aufnehmenden Gehäuses zusammengequetscht. Durch die Fortbewegung der Quetschrolle in Richtung der Spritzennadel wird das in der Ampulle enthaltene Medikament durch die Spritzennadel hinausgedrückt. Je nach der Geschwindigkeit, mit welcher die Quetschrolle vorwärtsbewegt wird, wird die durch die Spritzennadel ausgetragene Medikamentenmenge größer oder kleiner. Die Quetschrolle ist bei dieser Ausführung der Injektionsvorrichtung auf einem rotierenden Ring befestigt, der an einer Seite mit einer Verzahnung versehen ist, die mit einem durch einen Elektromotor angetriebenen Ritzel kämmt. Auch bei dieser Lösung ist die Handhabung der verformbaren Ampulle beim Einlegen in das Gehäuse und die Mengenüberwachung des durch die Spritzennadel ausgetragenen Medikamentes schwierig.

Bei einer anderen bekannten Injektionsvorrichtung - gemäß EP-A2 0 178 371 - ist eine Faltenbalgampulle ebenfalls in einem zylinderförmigen Gehäuse angeordnet, wobei ein Außendurchmesser und eine Höhe der Faltenbalgampulle im wesentlichen einem Innendurchmesser und einer Innenhöhe eines rohrförmigen Gehäuses entspricht. Eine Stirnseite des rohrförmigen Gehäuses ist mit einer Bodenplatte verschlossen. Von der anderen Stirnseite her kann ein Deckel in den Innenraum des rohrförmigen Gehäuses eingeschraubt werden. Zwischen der Bodenplatte und dem Deckel befindet sich die elastisch verformbare Ampulle. Durch das Einschrauben des Deckels in das rohrförmige Gehäuse wird das Medikament aus der Ampulle in die Injektionsnadel verdrängt. Auch bei dieser Ausführungsform der Injektionsvorrichtung erfordert das Einlegen der verformbaren Ampulle ein sehr genaues Arbeiten und die Dosiergenauigkeit reicht in vielen Fällen nicht aus.

Der vorliegenden Erfindung liegt die Aufgabe zugrunde, eine Injektionsvorrichtung zu schaffen, bei welcher auch bei Verwendung von verformbaren Ampullen die Handhabung derselben einfach möglich ist und die darüber hinaus einfach zu bedienen ist. Weiters soll ein gleichmäßiger Austrag des Medikamentes über die gesamte Abgabenmenge erzielt und eine Beschädigung der verformbaren Ampulle während des Auspressens verhindert werden.

Diese Aufgabe der Erfindung wird durch die im Kennzeichenteil des Patentanspruches 1 angegebenen Merkmale gelöst. Die Vorteile dieser Losung liegen darin, daß unabhängig von den Druckverhältnissen im Innenraum des Ampullenkörpers nur eine bestimmte vordefinierte Medikamentenmenge in der Zeiteinheit aus der Ampulle hinausgepreßt werden kann. Dadurch wird eine Fehldosierung ausgeschaltet und die Dosierung kann in einfacher Weise durch Abänderung des Drosselkanales an die in unterschiedlichen Fällen benötigte Medikamentenmenge angepaßt werden. Gleichfalls kann das in der Ampulle enthaltene Medikament immer in Richtung der kombinierten Verschluß- und Haltevorrichtung, also in Richtung des offenen Endes der Ampulle gepreßt werden. Ein unerwünschtes Austreten des Medikamentes aus der Ampulle in jenem Bereich, in welchem die Antriebsvorrichtung auf diese einwirkt, wird dadurch vermieden. Weiters wird durch die Verwendung eines Faltenbalges als Ampulle ein gleichmäßiger Austrag des Medikamentes erzielt und außerdem vermieden, daß sich die Ampulle zwischen der Antriebsvorrichtung und der Stützvorrichtung verklemmen kann.

Weiters kann dadurch die Positionierung der Stützvorrichtung oder die Führung der Antriebsvorrichtung zum Auspressen der Ampulle vereinfacht werden. Darüberhinaus kann der zum Verformen der Ampulle verwendete Teil ebenfalls in der Stützvorrichtung geführt werden, sodaß der Aufwand für die Gehäuse von Injektionsvorrichtungen bei Verwendung derartiger verformbarer Ampullen gering gehalten werden kann. Ebenso kann die Stützvorrichtung nicht nur zum Führen und Abstützen der Außenwände der verformbaren Ampulle, sondern gleichzeitig auch zur Führung des diese Ampulle verformenden Kolbens verwendet werden. Gleichzeitig wird damit sowohl eine Beschädigung der Ampulle während des Verformens als auch das Einwirken von radialen Kräften verhindert.

Es ist aber auch eine Ausbildung nach Patentanspruch 2 von Vorteil. Damit wird für die Herstellung des Ampullenkörpers ein kostengünstig und für hohe Stückzahlen maßgenau zu fertigender Grundwerkstoff verwendet.

Von Vorteil ist auch eine Weiterbildung nach Patentanspruch 3, da dadurch eine entsprechende Dichtheit der Ampulle erreicht wird.

Möglich ist auch eine Ausbildung nach Patentanspruch 4, da dadurch der Ampullenkörper durch die Haltekräfte der Ampulle in der Haltevorrichtung, insbesondere beim Einsetzen der Spritzennadel und beim Auspressen des Medikamentes, nicht belastet wird, sondern diese Stützkräfte von der Verschluß- und Haltevorrichtung übernommen werden.

Von Vorteil ist auch eine Ausführung nach Patentanspruch 5, wodurch eine Abstützung der Stützvorrichtung in Auspreßrichtung des Medikamentes ausreicht und die Injektionsvorrichtung bzw. deren Gehäuse einfach aufgebaut und das Einlegen der Ampulle erleichtert werden kann.

Eine weitere vorteilhafte Ausbildung ist im Patentanspruch 6 beschrieben, wodurch in vorteilhafter Weise die Verschluß- und Haltevorrichtung gleichzeitig als Originalitätsverschluß eingesetzt werden kann, sodaß ein zusätzlicher Originalitätsverschluß eingespart werden kann.

Von Vorteil ist eine Weiterbildung nach Patentanspruch 7, wodurch die Spritzennadel erst unmittelbar vor dem Auspressen des Medikamentes mit der Ampulle verbunden werden muß.

Es ist aber auch eine Fortbildung nach Patentanspruch 8 möglich, da dadurch eine sichere Halterung des Nadelträgers und eine dichte Verbindung zwischen der Ampulle und der Spritzennadel erreicht wird.

Eine vorteilhafte Weiterbildung ist im Patentanspruch 9 gekennzeichnet. Damit wird sichergestellt, daß das in der Ampulle enthaltene Medikament bis zum Gebrauch sicher verwahrt ist.

Es ist aber auch eine Ausbildung nach Patentanspruch 10 möglich, wodurch bei Verwendung von Faltenbalgampullen mit kreisförmigen Querschnitt eine Führung mit geringer Reibung möglich ist und die mit derartigen Stützvorrichtungen versehenen Ampullen auch als Ersatz der, ebenfalls mit runden Querschnitt versehenen Glasampullen verwendet werden können.

Vorteilhaft ist weiters eine Ausführung nach Patentanspruch 11, da dadurch eine Führung der verformbaren Ampulle über den gesamten Umfang möglich ist, sodaß unerwünschte Verformungen, die zu Verklemmungen der Ampulle in der Stützvorrichtung führen könnten, mit Sicherheit vermieden werden.

Eine andere Ausführungsvariante gemäß Patentanspruch 12 ist von Vorteil, weil dadurch auch Ampullen mit einer dünneren Wandstärke des Ampullenkörpers verwendet werden können, da ein Ausweichen der Ampulle in radialer Richtung durch die Stützvorrichtung zuverlässig verhindert ist.

Von Vorteil ist auch eine Ausbildung nach Patentanspruch 13, wodurch die Antriebsvorrichtung, beispielsweise ein die Ampulle zusammenpressender Kolben, über den gesamten Hub, über welchen die Ampulle zusammengepreßt wird, in einer exakten Lage relativ zu dieser geführt werden kann.

Vorteilhaft ist aber auch eine Weiterbildung nach Patentanspruch 14, da dadurch das Zusammendrücken der Ampulle durch einen geraden, sich über die Stützvorrichtung hinaus erstreckenden Balken erfolgen kann, der beidseits der Stützvorrichtung geführt bzw. mit Zug- oder Druckkräften beaufschlagt werden kann.

Weiters ist auch eine Ausbildung nach Patentanspruch 15 möglich, wodurch beim Einsetzen der Spritzennadel gleichzeitig die Sperrvorrichtung der Ampulle geöffnet werden kann.

Vorteilhaft ist auch eine Ausführungsvariante nach Patentanspruch 16, wodurch die Bewegung beim Einsetzen der Spritzennadel gleichzeitig zum Öffnen der Sperrvorrichtung herangezogen werden kann, andererseits jedoch die Spritzennadel auch in radialer Richtung verlaufen kann, um vor allem bei Injektionsvorrichtungen, die am Körper eines Menschen über längere Zeitdauer fixiert werden, eine entsprechende raumsparende Anordnung zu ermöglichen.

Eine mögliche vorteilhafte Ausbildung gemäß Patentanspruch 17 erreicht man vor allem auch dann, wenn die Spritzennadel senkrecht zur Längsachse der Ampulle angeordnet ist.

Es ist aber auch eine Weiterbildung nach Patentanspruch 18 möglich. Dadurch ist ein Einlegen der Stützvorrichtung bzw. der Ampulle in das Gehäuse durch den Kolben der Antriebsvorrichtung nicht behindert.

Weiters ist auch eine Ausbildung nach Patentanspruch 19 möglich, wodurch eine zentrale Verformung der Ampulle erreicht wird.

Von Vorteil ist aber auch eine Weiterbildung nach Patentanspruch 20, da dadurch das Einlegen der Ampulle bzw. der Stützvorrichtung erleichtert wird, vor allem dann, wenn ein Federantrieb oder dgl. verwendet wird.

Vorteilhaft ist aber auch eine Ausbildung nach Patentanspruch 24, da dann die Ampulle von oben her in das Gehäuse eingelegt und ordnungsgemäß positioniert werden kann, worauf dann das Gehäuse verschlossen werden kann.

Eine vorteilhafte Weiterbildung beschreibt Patentanspruch 25, da dadurch in jedem Fall verhindert wird, daß die Öffnungsvorrichtung auf der dem Kolben zugewandten Seite die Ampulle beschädigt.

Es ist aber auch eine Weiterbildung nach Patentanspruch 26 möglich. Dadurch ist auch eine automatische Betätigung der Antriebsvorrichtung möglich.

Weiters ist auch eine Ausführungsform nach Patentanspruch 27 vorteilhaft, wodurch eine möglichst spielfreie und kontinuierliche Beaufschlagung der Ampulle erzielt wird.

Von Vorteil ist aber auch eine Ausbildung nach Patentanspruch 28, da durch entsprechende Veränderung der Druckverhältnisse eines Fluides eine Veränderung der Ausstoßmenge des Medikamentes in einfacher Weise erzielt werden kann.

Eine besonders bevorzugte weitere Ausführungsform ist im Patentanspruch 29 gekennzeichnet. Damit kann die Tätigkeit der Antriebsvorrichtung sowie die ausreichende Abgabe des Medikamentes überwacht und bei Störungen in der Abgabe diese sofort optisch bzw. akustisch ersichtlich gemacht werden. Darüberhinaus kann dem Benutzer einer Injektionsvorrichtung in einfacher Weise signalisiert werden, daß eine neue Ampulle eingesetzt werden muß, sodaß medizinische Nachteile für den Patienten mit Sicherheit ausgeschaltet sind.

Eine andere vorteilhafte weitere Ausgestaltung ist im Patentanspruch 30 beschrieben. Dadurch kann die Abgabe des Medikamentes aus der Ampulle an die medizinischen Daten des Patienten angepaßt werden.

Vorteilhaft ist weiters eine Ausbildung nach Patentanspruch 31, da dadurch die Medikamentenmenge an die Aktivität des Patienten angepaßt werden kann, sodaß diesem bei höherer Aktivität gegebenenfalls mehr oder weniger von dem Medikament zugeführt werden kann.

Es ist aber auch eine Weiterbildung, wie sie nach Patentanspruch 32 beschrieben ist, möglich. Dadurch ist es in einfacher Weise möglich, die sehr dünnen Kanäle mit winkeligen Umlenkungen herzustellen.

Eine andere Ausführungsvariante ist im Patentanspruch 33 gekennzeichnet, wodurch das Eindringen von Kleinstteilen in die Spritzennadel und somit in den Körper eines Patienten einfach verhindert wird. Durch die Verwendung des Mikrofilters zum Abdichten des Drosselkanals kann dieser in mehrfacher Weise genutzt werden.

Eine vorteilhafte weitere Ausbildung ist im Patentanspruch 34 beschrieben. Dadurch wird die Herstellung des Drosselkanals vereinfacht und es kann auf kleiner Fläche eine relativ große Kanallänge untergebracht werden.

Weiters ist eine vorteilhafte Weiterbildung gemäß Patentanspruch 35 möglich, da auch dadurch scharfe Umlenkungen im Drosselkanal vermieden werden können.

Vorteilhaft ist auch eine andere Ausführungsvariante, wie sie im Patentanspruch 42 beschrieben ist, wodurch der Mikrofilter nur aus wenigen Teilen besteht.

Von Vorteil ist auch eine Weiterbildung, wie sie im Patentanspruch 43 beschrieben ist. Dadurch kann die zur Halterung der Filtermembran dienende Klemmscheibe gleichzeitig auch zu deren Befestigung in dem Haltering verwendet werden.

Weitere vorteilhafte Ausführungsformen der Erfindung sind in den weiteren Unteransprüchen gekennzeichnet.

Zum besseren Verständnis der Erfindung wird diese nachfolgend anhand der in den Zeichnungen gezeigten Ausführungsbeispiele näher erläutert.

Es zeigen:
- Fig.1: eine erfindungsgemäße Injektionsvorrichtung in vereinfachter schaubildlicher Darstellung, teilweise geschnitten;
- Fig.2: eine erfindungsgemäße Ampulle für eine erfindungsgemäße Injektionsvorrichtung in Seitenansicht geschnitten, gemäß den Linien II-II in Fig.3;
- Fig.3: die Ampulle nach Fig.2 in Stirnansicht;
- Fig.4: eine Ausführungsvariante einer erfindungsgemäßen zweiteiligen Injektionsvorrichtung im aufgeklappten Zustand und in Draufsicht;
- Fig.5: die Injektionsvorrichtung nach Fig.4 in Seitenansicht und geschnitten, gemäß den Linien V-V in Fig.6;
- Fig.6: die Injektionsvorrichtung im Schnitt, gemäß den Linien VI-VI in Fig.5;
- Fig.7: eine Ausführungsvariante einer erfindungsgemäßen Ampulle in Seitenansicht, geschnitten;
- Fig.8: eine Ausführungsvariante einer erfindungsgemäßen Ampulle mit einer einen quadratischen Querschnitt aufweisenden Stützvorrichtung, angeordnet in einer erfindungsgemäßen Injektionsvorrichtung, in Seitenansicht, geschnitten;
- Fig.9: ein Schaltschema zur Betätigung der Antriebsvorrichtung der erfindungsgemäßen Injektionsvorrichtung in Fig.8 in schaubildlicher Darstellung;
- Fig.10: eine weitere Ausführungsform einer erfindungsgemäßen Ampulle mit einem dieser zugeordneten Nadelträger in schematischer Darstellung und in Seitenansicht, teilweise geschnitten;
- Fig.11: eine andere Ausführung einer erfindungsgemäßen Injektionsvorrichtung in Seitenansicht, teilweise geschnitten;
- Fig.12: einen Teil einer erfindungsgemäßen Injektionsvorrichtung in Seitenansicht im Schnitt;
- Fig.13: die Injektionsvorrichtung nach Fig.12 in Stirnansicht geschnitten gemäß den Linien XIII-XIII in Fig.12;
- Fig.14: einen Nadelträger einer Injektionsvorrichtung mit integriertem Drosselkanal und Mikrofilter in Seitenansicht geschnitten;
- Fig.15: den Nadelträger nach Fig.14 in Stirnansicht geschnitten gemäß den Linien XV-XV in Fig.14;
- Fig.16: einen Mikrofilter einer Injektionsvorrichtung in Seitenansicht geschnitten;
- Fig.17: den Mikrofilter nach Fig.16 in Stirnansicht im Schnitt gemäß den Linien XVII-XVII in Fig.16;
- Fig.18: die Teile des Mikrofilters während des Einsetzens der Filtermembran in den Mikrofilter in Seitenansicht geschnitten;
- Fig.19: die Teile des Mikrofilters in ihrer Stellung unmittelbar nach dem Einsetzen der Filtermembran in den Mikrofilter in Seitenansicht und geschnitten;
- Fig.20: eine Injektionsvorrichtung mit einer Ausführungsvariante für die Arretiervorrichtung in schaubildlicher Darstellung, bei geöffnetem Deckel und ohne Ampulle;
- Fig.21: die Injektionsvorrichtung nach Fig.20 mit bei geöffnetem Deckel eingelegter Ampulle in schaubildlicher Darstellung;
- Fig.22: die Injektionsvorrichtung nach Fig.20 mit geschlossenem Deckel in schaubildlicher Darstellung;
- Fig.23: einen Teil der Injektionsvorrichtung nach den Fig.20 bis 22 in Stirnansicht, geschnitten;
- Fig.24: eine andere Ausführungsvariante einer Injektionsvorrichtung für den Einmalgebrauch in schaubildlicher Darstellung;
- Fig.25: einen Teil der Injektionsvorrichtung nach Fig.24 mit für die Medikamentenabgabe aktivierter Ampulle;
- Fig.26: eine andere Ausführungsvariante einer Ampulle im Transportzustand in Draufsicht, geschnitten;
- Fig.27: die Ampulle nach Fig.26 in aktivierter Stellung;
- Fig.28: die Drosselvorrichtung der in den Fig.26 und 27 dargestellten Ampulle in Draufsicht;
- Fig.29: einen Teil des Ampullenkörpers der Ampulle nach den Fig.26 und 27 in Draufsicht, geschnitten;
- Fig.30: eine andere Ausführungsform einer Ampulle in Draufsicht, geschnitten;
- Fig.31: die Ampulle nach Fig.30 mit einem Teil eines Gehäuses der Injektionsvorrichtung in zur Medikamentenabgabe aktivierten Stellung in Draufsicht und geschnitten.

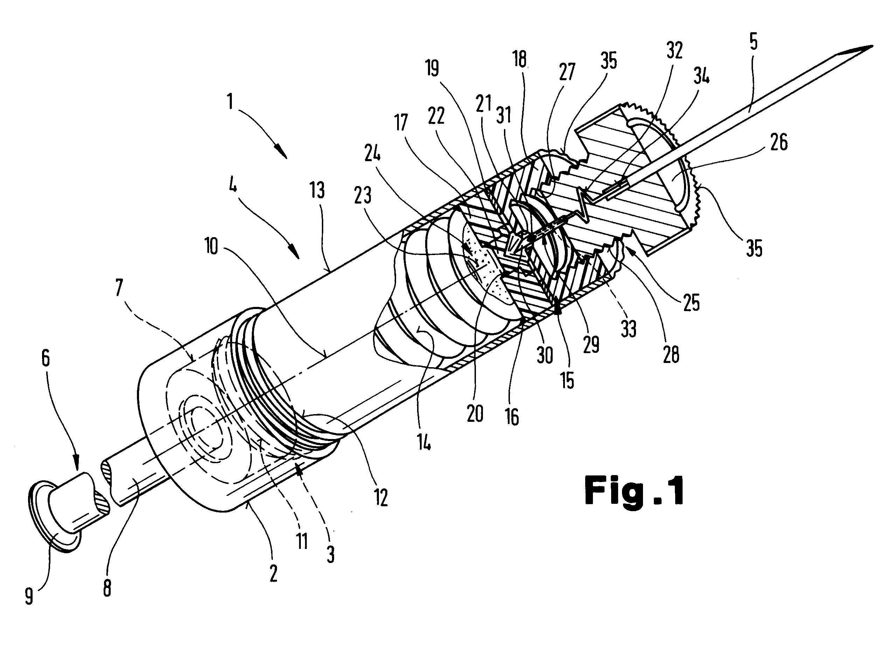

In Fig.1 ist eine Injektionsvorrichtung 1 gezeigt. Diese besteht aus einem Gehäuse 2 mit einer Halterung 3 für eine Ampulle 4, der in ihrem einen Stirnendbereich eine Spritzennadel 5 und in ihrem anderen Stirnendbereich eine Antriebsvorrichtung 6 zugeordnet ist. Die Antriebsvorrichtung 6 umfaßt einen Kolben 7, der im Gehäuse 2 geführt ist und über eine Kolbenstange 8, die mit einem Handgriff 9 versehen ist, entlang einer Längsachse 10 der Ampulle 4 in Richtung der Spritzennadel 5 verschiebbar ist. Die Halterung 3 des Gehäuses 2 weist ein in einer Bohrung angeordnetes Gewinde 11 auf, in welches die Ampulle 4 mit einem äußeren Gewinde 12 eingeschraubt ist. Dieses äußere Gewinde 12 ist auf einer Stützvorrichtung 13, die rohrförmig ausgebildet ist, angeordnet. In dieser Stützvorrichtung 13 ist beim dargestellten Ausführungsbeispiel ein durch einen Faltenbalg gebildeter Ampullenkörper 14 angeordnet. Eine mit dem Ampullenkörper verbundene Verschluß- und Haltevorrichtung 15 ist mit der Stützvorrichtung 13 beispielsweise durch eine Schweißung 16, z.B. eine Ultraschallschweißung, verbunden. Die Verschluß- und Haltevorrichtung besteht aus zwei Teilen 17,18, die untereinander ebenfalls über eine Kleberschicht 19 oder durch eine Schweißung verbunden sein können. Die beiden Teile 17 und 18 weisen Bohrungen 20,21 auf, die coaxial zur Längsachse 10 angeordnet sind. Zwischen den beiden Teilen 17,18 ist als Sperrvorrichtung 22 ein Diaphragma bzw. eine Membranscheibe z.B. aus Pharmagummi oder dgl. als Dichtscheibe angeordnet, die den Austritt eines schematisch angedeuteten Medikamentes 23 aus einem Innenraum 24 der Ampulle 4 verhindert. Weiters ist der Teil 18 über eine Kupplungsvorrichtung 25 mit einem Nadelträger 26, der die Spritzennadel 5 lagert, verbunden. Ein im Teil 18 angeordneter Kupplungsteil 27 wird durch ein Innengewinde und ein diesem zugeordneter Kupplungsteil 28 des Nadelträgers 26 durch ein Außengewinde gebildet. Über den Nadelträger steht in Richtung der Ampulle 4 eine Öffnungsvorrichtung 29 vor, die eine Spitze 30 zum Durchtrennen der Sperrvorrichtung 22 sowie eine Öffnung 31 zum Verbinden des Innenraumes 24 der Ampulle 4 mit der Spritzennadel 5 aufweist. Wird der Nadelträger 26 in den Teil 18 der Verschluß- und Haltevorrichtung 15 bis zum Anschlag eingedreht, so durchstößt die Spitze 30 die durch die Dichtscheibe gebildete Sperrvorrichtung 22 und tritt so weit in den Innenraum 24 der Ampulle bzw. der mit dieser verbundenen Bohrung 20 des Teiles 17 der Verschluß- und Haltevorrichtung 15 ein, sodaß das Medikament 23 durch die Öffnung 31 zur Spritzennadel 5 gelangen kann.

Ist die als Originalitätsverschluß wirkende Sperrvorrichtung 22 geöffnet, so kann durch eine Verschiebung des Kolbens 7 in Richtung der Spritzennadel 5 die aus verformbaren Kunststoffmaterial, beispielsweise Pharmagummi, Silikon oder dgl., bestehende Ampulle 4 bzw. deren Ampullenkörper 14, der als Faltenbalg ausgebildet ist, zusammengepreßt und das Medikament 23 durch die Spritzennadel 5 ausgepreßt werden.

Bevorzugt ist der Ampullenkörper 14 einstückig mit dem Teil 17 ausgebildet und besteht aus einem elastisch verformbaren Kunststoff, beispielsweise Silikon, während der Teil 18 aus einem stabileren formfesten Kunststoff, beispielsweise PVC oder dgl., bestehen kann, da dieser mit dem Medikament nicht in Berührung kommt. Durch die höhere Festigkeit und Steifigkeit des Teiles 18 bildet dieser ein besseres Widerlager für den Nadelträger 26 sowohl beim Einstechen der Spritzennadel 5 als auch beim Durchtrennen der Sperrvorrichtung 22.

Um die Dosierung des Medikamentes 23 beim Auspressen aus dem Innenraum 24 der Ampulle 4, unabhängig vom durch die Antriebsvorrichtung 6 ausgeübten Druck, zu ermöglichen, ist im Nadelträger 26 ein Drosselkanal 32 angeordnet, der an eine mit einer Öffnung 31 verbundene Bohrung 33 der Öffnungsvorrichtung 29 anschließt und diese mit einem Spritzennadelkanal 34 der Spritzennadel 5 verbindet. Je nach dem Querschnitt des Drosselkanales 32, der beispielsweise 0,02mm Durchmesser aufweisen kann, wird die in der Zeiteinheit abgebbare Medikamentenmenge auf einen bestimmten Wert begrenzt.

Um das Einschrauben des Nadelträgers 26 in den Teil 18 zu erleichtern, kann der Nadelträger, wie schematisch angedeutet, mit einer Riffelung 35 versehen sein. Gleichermaßen kann auch die Stützvorrichtung 13 im Bereich des Teiles 18 mit einer Riffelung 35 ausgestattet werden.

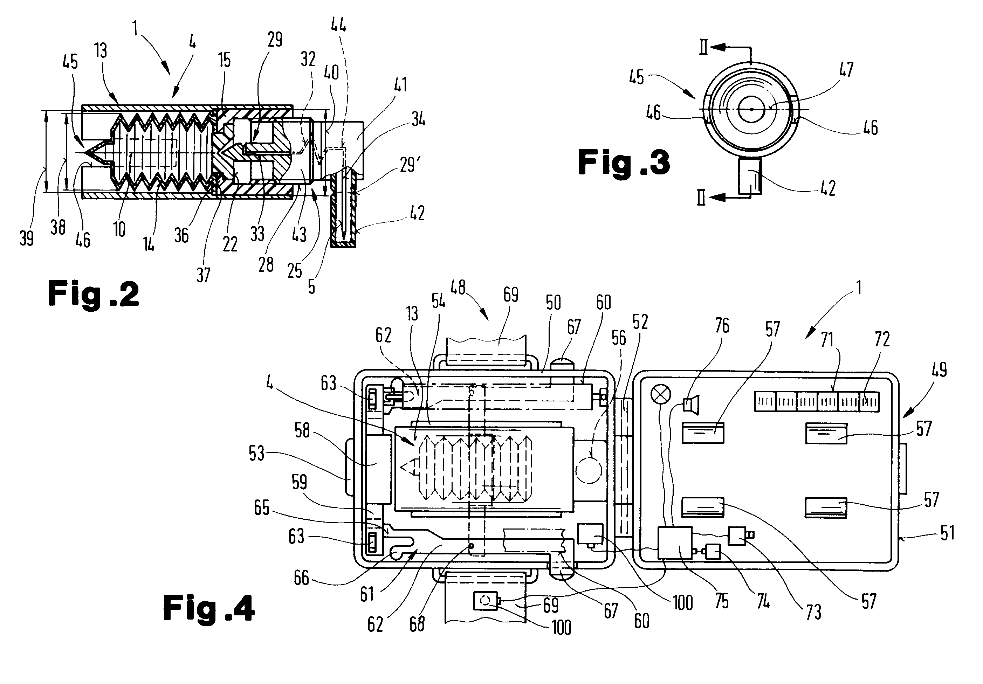

In den Fig.2 bis 6 ist eine andere Ausführungsform einer Injektionsvorrichtung 1 und einer Ampulle 4 gezeigt. Diese besteht aus einer rohr- bzw. hülsenförmigen Stützvorrichtung 13, der Verschluß- und Haltevorrichtung 15, die im vorliegenden Ausführungsbeispiel einteilig ausgebildet ist, einer durch eine Membran gebildeten Sperrvorrichtung 22, die beispielsweise aus Pharmagummi bestehen kann sowie dem Ampullenkörper 14, der durch einen Faltenbalg gebildet ist. Der Faltenbalg besteht aus einem elastischen, für die Aufbewährung von Medikamenten zugelassenen Kunststoff, beispielsweise einem Silikonkunststoff, und weist an seinem der Verschluß- und Haltevorrichtung 15 zugewandten Ende einen umlaufenden Flanschring 36 auf. Der Flanschring 36 ist mit einer Stirnfläche 37 der Verschluß- und Haltevorrichtung 15, die aus einem formfesten Kunststoff besteht, z.B. PVC, verklebt bzw. verschweißt. Der Flanschring 36 kann, wie beim dargestellten Ausführungsbeispiel gezeigt, einen geringfügig größeren Außendurchmesser aufweisen als ein Außendurchmesser 38 des Ampullenkörpers 14 bzw. ein Innendurchmesser 39 der Stützvorrichtung 13 im Bereich des Ampullenkörpers 14, der geringfügig größer ist als der Außendurchmesser 38 des Ampullenkörpers 14. Gleichfalls kann die Verschluß- und Haltevorrichtung 15 einen äußeren Durchmesser 40 aufweisen, der geringfügig größer ist als der Innendurchmesser 39. Dadurch kann die Verschluß- und Haltevorrichtung 15 exakt in der Stützvorrichtung 13 positioniert werden. Ein Nadelträger 41 ist mit einer senkrecht zur Längsachse 10 der Ampulle 4 ausgerichteten Spritzennadel 5 versehen. Zum Schutz der Spritzennadel 5 vor der Inbetriebnahme der Injektionsvorrichtung 1 ist die Spritzennadel 5 mit einer Nadelschutzkappe 42 versehen. Zwischen der Nadelschutzkappe 42 und dem Nadelträger 41 kann eine Öffnungseinrichtung 29' angeordnet sein, die beispielsweise entsprechend den Ausführungen in der WO-A186/03126 des gleichen Anmelders ausgebildet sein kann. Zwischen dem Nadelträger 41 und der Ampulle 4 ist ein Zwischenstück 43 angeordnet. In dem Zwischenstück 43 ist der Drosselkanal 32 angeordnet, der in einen Kanal 44 im Nadelträger 41 mündet, der seinerseits mit dem Spritzennadelkanal 34 der Spritzennadel 5 verbunden ist. Der Einlaß des Drosselkanales 32 ist mit der Bohrung 33 in der durch eine spitze Nadel gebildeten Öffnungsvorrichtung 29 verbunden, die in die Öffnung 31 dieser Nadel mündet.

Bei diesem Ausführungsbeispiel bilden das Zwischenstück 43 und der Nadelträger 41 einen gemeinsamen Bauteil. Es ist aber auch möglich, daß diese beiden Teile über einen Bajonettverschluß oder dgl. verbunden sind und getrennte Teile bilden. Der Kupplungsteil 28 der identisch zur Darstellung in Fig.1 ausgebildeten Kupplungsvorrichtung 25 ist, ist am Zwischenstück 43 angeordnet.

Wie aus dieser Darstellung weiters zu ersehen ist, ist die Stützvorrichtung 13 mit einer Führungsbahn 45 versehen, die durch Längsschlitze 46 gebildet ist.

Wie besser aus Fig.3 zu ersehen ist, sind die die Führungsbahn 45 bildenden Längsschlitze 46 auf einer Diametralen 47 angeordnet. Diese sich parallel zur Längsachse 10 erstreckende Führungsbahn 45 dient, wie anhand der Fig.4 bis 6 erläutert werden wird, zur besseren Führung der auf den Ampullenkörper 14 einwirkenden Antriebsvorrichtung.

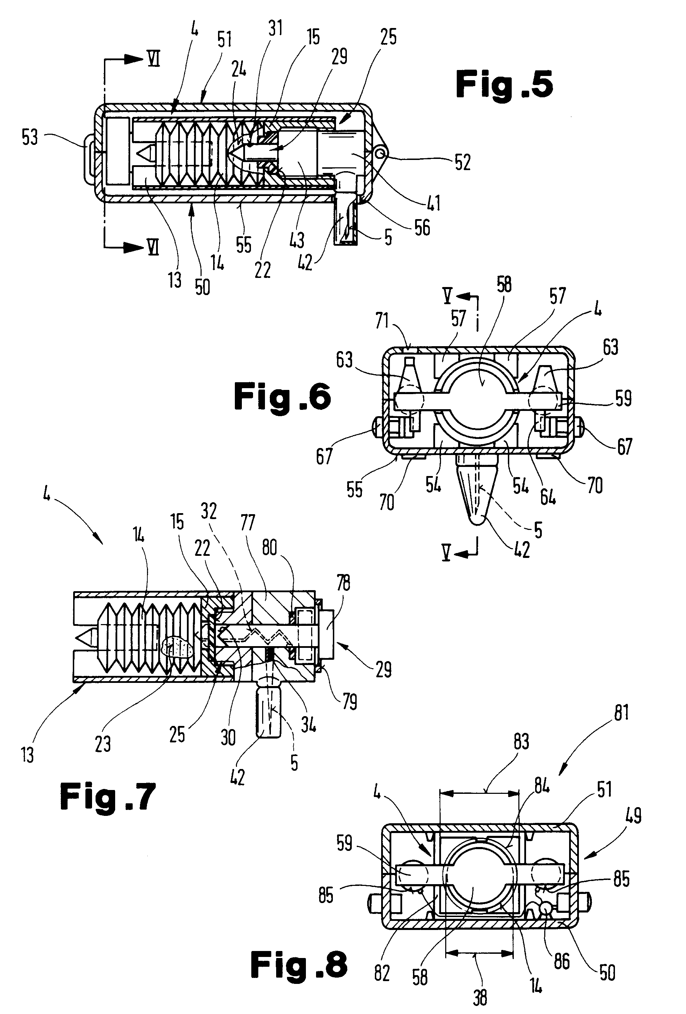

In den Fig.4 bis 6 ist zur Aufnahme der in Fig.2 und 3 dargestellten Ampulle 4 eine Injektionsvorrichtung 48 gezeigt, die aus einem Gehäuse 49 besteht, welches einen unteren Gehäuseteil 50 und einen oberen Gehäuseteil 51 aufweist, die über ein Scharnier 52 gelenkig miteinander verbunden sind. Die Verbindung der beiden Gehäuseteile 50,51 im geschlossenen Zustand erfolgt mit einer schematisch angedeuteten Schnappvorrichtung 53. In den unteren Gehäuseteil 50 wird die Ampulle 4 zwischen Führungsleisten 54 eingelegt. Wie ersichtlich, ist beim Einlegen der Ampulle 4 der Nadelträger 41 sowie das Zwischenstück 43 mit dieser gekuppelt. Dazu sind diese beiden Teile so weit in die Kupplungsvorrichtung 25 eingedreht, daß die Öffnungsvorrichtung 29 die Sperrvorrichtung 22, also die Membran, durchstoßen hat und sich die Öffnung 31 - wie aus Fig.5 ersichtlich - im Innenraum 24 des Ampullenkörpers 14 befindet. Beim Einsetzen der Ampulle 4 wird die Nadelschutzkappe 42 durch eine in einer Bodenplatte 55 angeordnete Öffnung 56 hindurchgeführt, sodaß die Spritzennadel 5 über die Bodenplatte 55 vorragt. Nachdem die Ampulle 4 derart positioniert ist, wird der obere Gehäuseteil 51 zugeklappt, wodurch die Ampulle 4 mit den auf diesem Gehäuseteil 51 angeordneten Führungsleisten 57 auch von oben her in ihrer Lage fixiert wird. Wie aus den Fig.4 und 6 besser zu ersehen ist, ist dem von der Verschluß- und Haltevorrichtung abgewendeten Ende der rohrförmigen Stützvorrichtung 13 ein Kolben 58 zugeordnet, der auf einem Querbalken 59 gelagert ist. Der Querbalken 59 ist mit beidseits der Ampulle 4 angeordneten Federn 60 einer Antriebsvorrichtung 61 verbunden, die auf ihrem von den Querbalken 59 abgewendeten Ende am Gehäuseteil 50 befestigt sind. Die Federn 60 können beispielsweise durch Schraubenfedern gebildet sein. Der Querbalken 59 wird durch Arretierbügeln 62, die ebenfalls Bestandteil der Antriebsvorrichtung 61 sind, in seiner in Fig.4 in vollen Linien gezeichneten Stellung gehalten. Wie besser aus Fig.6 zu ersehen ist, weist der Querbalken Mitnehmer 63 auf, mit denen er in Richtung der in vollen Linien gezeigten Stellung entgegen der Wirkung der Federn 60 bewegt werden kann. Dabei wird durch Haltezapfen 64 - Fig.6 - ein Rastarm 65 des Arretierbügels 62 entgegen der Wirkung eines Federarmes 66 in eine entgegen der Ampulle 4 gerichtete Richtung verformt. Nachdem der Haltezapfen 64 seine Endstellung erreicht hat, kann der Rastarm 65 in die in Fig.4 in vollen Linien gezeichnete Stellung zurückfedern und arretiert den Querbalken 59 entgegen der Wirkung der Federn 60 in seiner in vollen Linien gezeichneten Stellung.

Ist nun der obere und untere Gehäuseteil 50,51 geschlossen, kann die Antriebsvorrichtung 61 dadurch aktiviert werden, daß die beidseits des unteren Gehäuseteiles 50 über diesen vortagenden Druckknöpfe 67 in Richtung der Ampulle 4 gedrückt werden, wodurch der Arretierbügel 62 um einen Schwenkpunkt 68 verformt wird und der Rastarm 65 den Haltezapfen 64 freigibt, sodaß durch die Wirkung der Federn 60 der Querbalken 59 in Richtung der Öffnung 56 bzw. der Verschluß- und Haltevorrichtung 15 verstellt werden kann. Durch den mit dem Kolben 58 über die Federn 60 ausgeübten Druck wird nun das in der Ampulle 4 enthaltene Medikament 23 in Richtung der Spritzennadel 5 gepreßt. Nachdem die Nadelschutzkappe 42 entfernt ist, kann sich der Benutzer dieser Injektionsvorrichtung die Spritzennadel 5 z.B. in den Unterarm stechen und das Gehäuse z.B. mit einem schematisch angedeuteten Band 69, ähnlich einem Uhrband oder einer Gummimanschette, am Körper z.B. am Arm oder Bein fixieren. Durch die Wirkung der Federn 60, in Verbindung mit dem Drosselkanal 32, wird über eine aufgrund der Federkraft und der in der Ampulle 4 vorhandenen Medikamentmenge vorgegebene Zeitspanne das Medikament gleichmäßig an den Patienten abgegeben. Diese Injektionsvorrichtung ist daher mit großem Vorteil bei jenen Patienten, wie Zuckerkranken, zu verwenden, die eine ständig gleichbleibende geringfügige Medikamentenmenge benötigen, um einen gewünschten therapeutischen Effekt zu erzielen.

Zur besseren Halterung der Injektionsvorrichtung 48 kann der untere Gehäuseteil 50 mit hautverträglichen Klebebändern 70 versehen sein, um bei Bewegungen des Patienten schmerzhafte Verlagerungen der Spritzennadel 5 auszuschalten.

Um den Patienten bzw. dem Pflegepersonal oder. dem Arzt jederzeit einen Überblick über die bereits verabreichte Menge an Medikament zu ermöglichen, kann das dem oberen Gehäuseteil 51 zugewandte Stirnende der Mitnehmer 63 färbig markiert sein und in einem Bewegungsbereich der Mitnehmer 63 ein Sichtfenster 71 angeordnet sein, welches, wie in Fig.4 schematisch angedeutet, mit einem Raster 72, der der Menge des abgegebenen bzw. der Menge des noch in der Ampulle 4 befindlichen Medikamentes entsprechen kann, angeordnet sein.

Ist die Ampulle 4 leer, d.h. hat der Querbalken 59 bzw. der Kolben 58 die in Fig.4 in strichlierten Linien schematisch angedeutete Endstellung erreicht, so ist eine neue Ampulle 4 einzusetzen. Dazu wird das Gehäuse 49 geöffnet, der Querbalken 59 mit den Mitnehmem 63 in seine in vollen Linien gezeigte Ausgangsstellung zurückbewegt und mittels der Arretierbügel 62 arretiert, worauf die Ampulle 4 entnommen und gegebenenfalls der Nadelträger 41 mit dem Zwischenstück 43 aus der Verschluß- und Haltevorrichtung 15 herausgedreht werden kann, um in eine neue Ampulle 4 eingesetzt zu werden.

Um zu verhindern, daß der Patient bzw. das Pflegepersonal oder der Arzt jenen Zeitpunkt übersieht, zu welchem das Medikament 23 zur Gänze entleert ist bzw. keine Medikamentenabgabe aus der Ampulle 4 mehr möglich ist, kann der Ampulle 4 oder dem Querbalken 59 z.B. im oberen Gehäuseteil 51 ein Endschalter 73 zugeordnet sein, der mit einer z.B. durch eine Batterie 74 gebildeten Energiequelle und einer Steuervorrichtung 75 sowie einem Summer 76 verbunden sein kann. Wird der Endschalter 73, der durch einen mechanisch betätigten Mikroschalter oder einen berührungslos arbeitenden Näherungsschalter auf elektromagnetischer Basis oder ähnlichem oder durch eine Lichtschranke gebildet sein kann, durch den Querbalken 59 berührt oder die Lichtschranke unterbrochen, so wird durch die Steuervorrichtung 75 der Summer 76 aktiviert und dem Träger einer derartigen Injektionsvorrichtung kann somit das Ende der Medikamentenabgabe akustisch oder, wie schematisch weiter angedeutet, durch eine kleine blinkende Leuchtdiode auch optisch angezeigt werden. Selbstverständlich ist die Anordnung einer derartigen Steuereinrichtung 75 mit Endschaltung nicht zwingend mötig, sie kann jedoch in Verbindung mit dem Sichtfenster 71 oder auch unabhängig von diesem in einer Injektionsvorrichtung 48 angeordnet sein.

Wie weiters in Fig.2 beispielsweise gezeigt, kann der Nadelträger 41 in einer zur Längsachse 10 verlaufenden Parallelebene geteilt sein. Die beiden Teile des Nadelträgers 41 werden im Zuge des Produktionsvorganges miteinander verklebt bzw. ultraschallverschweißt. Die Bohrung 33 kann dann je zur Hälfte in jeden der beiden Teile des Nadelträgers 41 angeordnet sein.

In Fig.7 ist eine andere Ausführungsvariante einer Ampulle 4 gezeigt. Diese weist wie bereits bei der in Fig.2 gezeigten und beschriebenen Ampulle 4 eine Stützvorrichtung 13 auf und einen durch einen Faltenbalg gebildeten Ampullenkörper 14. In der Verschluß- und Haltevorrichtung 15 der Ampulle 4 ist über eine Kupplungsvorrichtung 25 ein Nadelträger 77 befestigt. Dieser Nadelträger 77 weist eine Spritzennadel 5 auf, die innerhalb einer Nadelschutzkappe 42 angeordnet ist. Zum Durchtrennen einer durch eine Membran gebildeten Sperrvorrichtung 22 ist eine Öffnungsvorrichtung 29 vorgesehen, die aus einem Druckbolzen 78 besteht, der an seinem vorderen Ende mit einer Spitze 30 zum Durchtrennen der Membran der Öffnungsvorrichtung 29 versehen ist. Der Druckbolzen 78 kann in seiner in vollen Linien gezeichneten Stellung durch einen Abreißring 79 gesichert sein. Soll nun ein Medikament 23 aus der Ampulle 4 entnommen werden, so wird der Abreißring 79 entfernt, wodurch eine Längsbewegung des Druckbolzens 78 in Richtung des Ampullenkörpers 14 freigegeben wird. Die Spitze 30 durchstößt dann die Sperrvorrichtung 22 und das Medikament 23 kann in den Drosselkanal 32, der in der Oberfläche des Druckbolzens 78 vertieft angeordnet ist, zum Spritzennadelkanal 34 der Spritzennadel 5 durchfließen. Die Ausbildung des Drosselkanales 32 ist so gewählt, daß bei voll eingedrückter, in strichlierten Linien dargestellter Stellung des Druckbolzens 78 der Ausgang des Drosselkanales 32 mit dem Spritzennadelkanal 34 direkt gekuppelt ist.

Zwischen dem betätigungsseitigen Ende des Druckbolzens 78 und dem Nadelträger 77 kann eine weitere Dichtscheibe 80 angeordnet sein, um zu verhindern, daß gegebenenfalls das Medikament anstelle in den Spritzennadelkanal 34 in Richtung des betätigungsseitigen Endes des Spritzenkolbens austritt.

In Fig.8 ist eine Injektionsvorrichtung 81 gezeigt, die ähnlich wie die Injektionsvorrichtung in den Fig.2 bis 6 ein Gehäuse 49 aufweist, welches aus einem unteren Gehäuseteil 50 und einem oberen Gehäuseteil 51 besteht. In dem Gehäuseteil ist der Querbalken 59 mit dem Kolben 58 angeordnet. Wie ersichtlich, weist der Ampullenkörper 14 der Ampulle 4 einen runden Querschnitt auf, wogegen die diesen Ampullenkörper 14 aufnehmende Stützvorrichtung 82 einen etwa quadratischen Querschnitt aufweist. Selbstverständlich ist es auch möglich, diese mit einem rechteckigen oder mehreckigen Querschnitt zu versehen. Ein Innenmaß 83 der Stützvorrichtung 82 entspricht einem Durchmesser eines Hüllkreises 84, der die Innenseiten der Stützvorrichtung 82 tangiert und geringfügig größer ist als ein Außendurchmesser 38 des Ampullenkörpers 14. Zur Betätigung des Querbalkens 59 sind im gezeigten Ausführungsbeispiel fluidbetätigte Zylinderkolbenanordnungen 85 angeordnet, deren von der Ampulle 4 abgewendete Zylinderkammern mit einem Druckspeicher 86 für das Fluid verbunden sind. In diesem Druckspeicher 86 ist beispielsweise eine Gummiblase enthalten, die auf das Fluid einen voreinstellbaren Druck ausübt. Bei der Rückstellung des Querbalkens 59 in die in Fig.4 in vollen Linien gezeichnete Stellung wird das Fluid aus den Zylinderkolbenanordnungen 85 in den Druckspeicher 86 verdrängt und bewirkt nach Freigabe des Querbalkens 59 durch Verschwenken der Arretierbügel 62, die in gleicher Art wie zu den Fig.4 bis 6 beschrieben, angeordnet sein können, eine Verschiebung des Kolbens 58 in Richtung der Ampulle 4.

Selbstverständlich ist es aber auch möglich, in dem Gehäuse 49 der Injektionsvorrichtung 81 eine Luftpumpe anzuordnen, die über eine Batterie oder eine externe Stromquelle beaufschlagt werden kann. Durch entsprechende Steuerventile ist es möglich, den Querbalken 59 in der zum Auspressen des Medikamentes aus der Ampulle 4 notwendigen Richtung als auch zur Rückstellung in die Ausgangslage zu beaufschlagen.

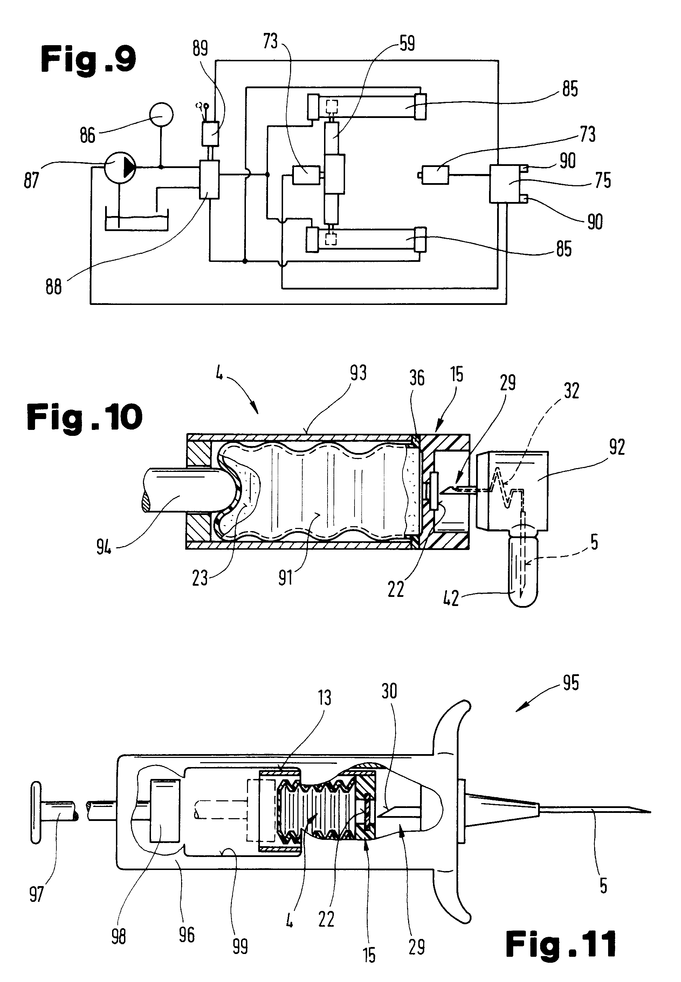

In Fig.9 ist ein schematisches Schaltbild einer Fluidanlage zum Betrieb der Zylinderkolbenanordnungen 85 dargestellt. In diesem Fall ist zusätzlich zum Druckspeicher 86 eine Fluidpumpe 87, beispielsweise für Hydrauliköl oder auch Luft, angeordnet. Über ein Steuerventil 88 kann das Druckmittel vom Druckspeicher 86 bzw. der Fluidpumpe 87 wahlweise in die durch die Kolben voneinander getrennten Zylinderkammern der beiden kolbenstangenlosen Zylinderkolbenanordnungen 85 eingepreßt werden. Die Kolben der beiden Zylinderkolbenanordnungen 85 sind durch den Querbalken 59 miteinander verbunden. Dem Querbalken 59 ist in jeder der beiden Endstellungen ein Endschalter 73 zugeordnet, der über eine Steuervorrichtung 75 mit dem Antrieb der Fluidpumpe 87 und einem Antrieb 89 für das Steuerventil 88 gekuppelt ist. Durch manuelle Betätigung des Steuerventiles 88 bzw. durch eine automatische Betätigung über die Steuervorrichtung 75, die über Befehlstasten 90 aktiviert werden kann, ist es möglich, die Bewegung der Zylinderkolbenanordnung 85 in der gewünschten Art und Weise zu steuern, sodaß bei Bewegung des Querbalkens 59 in Richtung des der Steuervorrichtung 75 näherliegenden Endschalters 73 das Medikament aus der Ampulle 4 hinausgepreßt wird, während durch gegengleiche Beaufschlagung der Querbalken 59 in seine in vollen Linien gezeichnete Ausgangsstellung zurückbewegt werden kann.

Selbstverständlich ist es möglich, die Steuervorrichtung 75 auch mit einer Überwachung des Füllzustandes der Ampulle 4 auszustatten, wie dies anhand der Fig.4 im Detail bereits beschrieben wurde.

In Fig.10 ist eine andere Ausführungsvariante einer Ampulle 4 gezeigt. Diese Ampulle umfaßt einen sackartigen Ampullenkörper 91 aus Silikon, der an seinem der Verschluß- und Haltevorrichtung 15 zugewandten Ende mit einem umlaufenden Flanschring 36 versehen ist. Die Verschluß- und Haltevorrichtung 15 wird durch eine aus hartem biegefesten Kunststoff bestehende scheibenartigen Teil gebildet, mit der der Flanschring 36 durch Schweißung, Klebung oder durch Klemm-Mittel verbunden ist. In der Verschluß- und Haltevorrichtung 15 ist eine Sperrvorrichtung 22, beispielsweise eine elastische Membran, angeordnet, die durch die Öffnungsvorrichtung 29 eines Nadelträgers 92 durchstochen werden kann. Der Nadelträger 92 ist, wie bereits bei den vorhergehenden Ausführungsbeispielen beschrieben, mit einer Spritzennadel 5 versehen, die in dem Nadelträger 92 eingespritzt sein kann. Desweiteren ist in diesem Nadelträger 92 auch der bereits in den vorhergehenden Ausführungsbeispielen beschriebene Drosselkanal 32 angeordnet.

Der sackartige Ampullenkörper 91 ist in eine Stützvorrichtung 93, die aus einem Rohrstück gebildet ist, eingeschoben und im Bereich des Flanschringes 36 mit dem Ampullenkörper 91 verbunden.

Das Ausbringen des in der Ampulle 4 enthaltenen Medikamentes 23, die transparent ausgebildet sein kann, um den Füllzustand jederzeit überprüfen zu können, erfolgt mittels eines Stößels 94, durch den der Ampullenkörper 91 umgestülpt wird, wobei dadurch das Medikament 23 durch die Spritzennadel 5 ausgepreßt wird.

In Fig.11 ist eine Injektionsvorrichtung 95 gezeigt, die ein Gehäuse 96 aufweist, in dem eine Kolbenstange 97 mit einem Kolben 98 geführt ist. Dieses ist mit einer Ausnehmung 99 versehen, deren Breite und Länge größer ist als ein Durchmesser bzw. eine Länge der Stützvorrichtung 13 der Ampulle 4. Nachdem die Ampulle 4 in die Ausnehmung 99 des Gehäuses 96 eingelegt worden ist, wird diese mit dem Kolben 98 in Richtung der Spritzennadel 5 in das Gehäuse 96 eingeschoben und läuft dabei auf eine Spitze 30 der durch eine Nadel gebildeten Öffnungsvorrichtung 29 auf, die den Originalitätsverschluß bzw. die Membran in der Verschluß- und Haltevorrichtung 15 durchtrennt, sodaß bei einem weiteren Vorschieben des Kolbens 98 in Richtung der Spritzennadel 5 das Medikament durch die Spritzennadel 5 ausgetragen werden kann. Das Gehäuse 96 kann, wie angedeutet, mit Gegenhaltern versehen sein, um eine bessere Abstützung der Injektionsvorrichtung 95 in der Hand zu ermöglichen.

Selbstverständlich ist es in Verbindung mit der Steuervorrichtung 75 auch möglich, wie beispielsweise im unteren Gehäuseteile 50 in Fig.4 bzw. in Band 69 schematisch angedeutet, Meßfühler 100 anzuordnen, die über Steuerleitungen mit der Steuervorrichtung 75 verbunden sein können. Über diese Meßfühler 100 ist es möglich, beispielsweise den Puls bzw. den Blutdruck oder sonstige Körperdaten, wie Temperaturen oder dgl., zu erfassen, um daraus über einen Rechner in der Steuervorrichtung 75 den Gesundheitszustand des Patienten überwachen zu können, sodaß gegebenenfalls in Abhängigkeit von diesen Daten bzw. Meßwerten die Medikamentenabgabe erhöht bzw. verringert werden kann. Für diese Meßfühler 100 können selbstverständlich alle beliebigen derzeit im Stand der Technik bekannten Fühler zur Feststellung der Körpertemperatur, des Pulsschlages bzw. des Blutdruckes herangezogen werden.

Im Rahmen der Erfindung ist es auch möglich, daß die Meßfühler 100 als Rüttelfühler ausgebildet sind, um die Bewegungen eines Patienten feststellen zu können. Dadurch ist es möglich, daß in der aktiven Tageszeit, vor allem wenn Sport betrieben wird oder sich der Patient mehr bewegt, eine höhere Medikamentenmenge an die Spritzennadel 5 abgegeben wird, während in Zeiten, wo der Patient ruht, beispielsweise während des Schlafes, eine geringere Menge an Medikament abgegeben wird. Desgleichen ist es natürlich auch möglich, entsprechende Rüttelantriebe, wie beispielsweise bei Uhren, zu verwenden, um die Antriebsvorrichtung zu aktivieren bzw. über die dadurch erzeugte Federkraft eine Pumpe oder andere Antriebselemente, wie Dynamo oder Federwerke, anzutreiben.

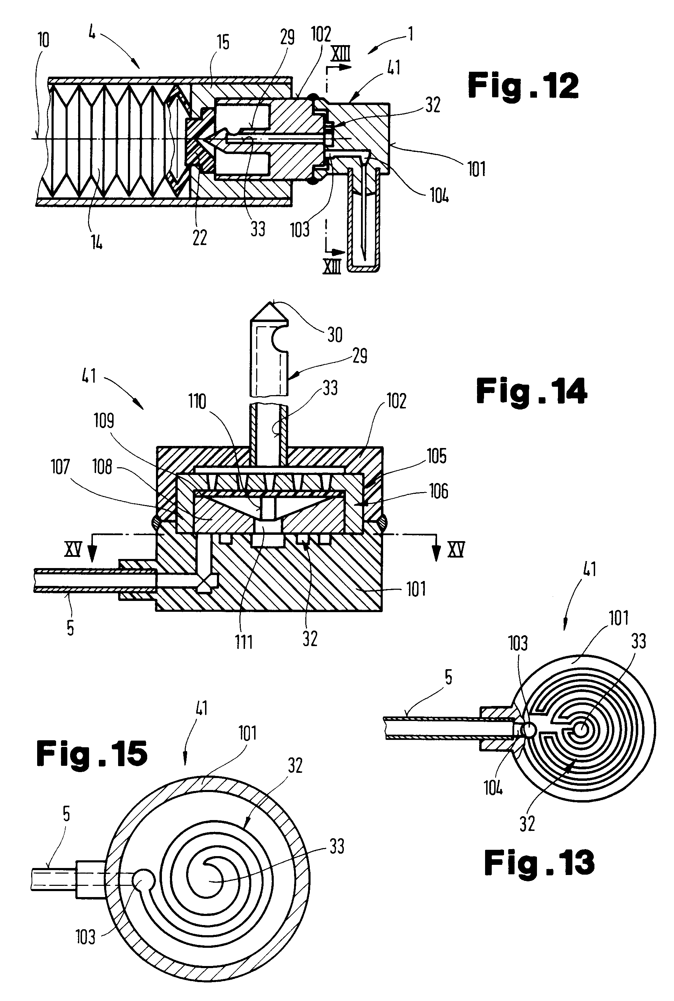

In Fig.12 ist von einer Injektionsvorrichtung 1 eine Ampulle 4 gezeigt, die beispielsweise entsprechend der Ampulle 4 in Fig.2 ausgebildet sein kann, weshalb für gleiche Teile gleiche Bezugszeichen verwendet werden. In einer Verschluß- und Haltevorrichtung 15 ist die durch eine Gummimembran gebildete Sperrvorrichtung 22 angeordnet. Die Verschluß- und Haltevorrichtung 15 ist mit dem Ampullenkörper 14 dicht verbunden. Zum Durchstoßen der Sperrvorrichtung 22 ist eine durch eine Nadelspitze gebildete Öffnungsvorrichtung 29 angeordnet. Diese ist ihrerseits in einem Nadelträger 41 eingespritzt bzw. einstückig mit diesem aus Kunststoff hergestellt. Eine Bohrung 33 ist mit einem Drosselkanal 32 verbunden. Zur Herstellung dieses Drosselkanals 32 besteht der Nadelträger 41 aus zwei Teilen 101, 102 die über eine Ultraschalschweißung miteinander verbunden sind.

Wie besser aus Fig.13 zu ersehen ist, ist im Teil 101 des Nadelträgers 41 der Drosselkanal 32 in Art einer Schlangenlinie angeordnet, wobei dieser aus mehreren konzentrisch zueinander verlaufenden Ringkanalteilen, die sich etwa über einen Winkelbereich von 320 Grad erstrecken besteht, die untereinander in radialer Richtung über Querkanäle verbunden sind. Der Ringkanal mit dem größten Durchmesser ist mit einer Auslaßbohrung 103 versehen, die mit einem in die Spritzennadel 5 mündenden Anschlußstutzen 104 verbunden ist. Durch die Anzahl der Ringkanalteile sowie der sich daraus ergebenden unterschiedlichen Länge des Drosselkanals 32 kann eine Zeitspanne innerhalb welcher das Medikament aus dem Ampullenkörper 14 in die Spritzennadel 5 austreten kann festgelegt werden. Demzufolge kann durch die Ausbildung des Drosselkanals 32 die Indikation der mit einer erfindungsgemäßen Injektionsvorrichtung abgegebenen Medikamentes festgelegt werden.

In Fig.14 ist eine andere Ausführungsvariante eines Nadelträgers 41 gezeigt, der ebenfalls aus zwei Teilen 101 und 102 besteht. Im Teil 101 ist wiederum ein Drosselkanal 32 angeordnet, der z.B. entsprechend der Darstellung in Fig.15 spiralförmig ausgebildet ist jedoch auch gemäß der Darstellung in Fig.13 ausgebildet sein kann. Es ist aber ebenso möglich, den Drosselkanal 32 im Teil 101 des Nadelträgers 41 in Art einer Zickzacklinie anzuordnen. Der Teil 102 ist mit der Öffnungsvorrichtung 29 und der Teil 101 mit der Spritzennadel 5 verbunden. Zwischen dem Auslaß der Bohrung 33 und dem Drosselkanal 32 ist ein Mikrofilter 105 angeordnet. Dieser soll verhindern, daß mit dem Medikament Schwebekörper oder beim Durchstechen der Membran der Sperrvorrichtung 22 mit der Spitze 30 der Öffnungsvorrichtung 29 entstandene Gummipartikel in die Spritzennadel 5 eintreten können. Außerdem soll dadurch verhindert werden, daß im Medikament während der Lagerung entstandene Kristalle in die Spritzennadel 5 gelangen können. Der Mikrofilter 105 besteht aus einem in den Teil 102 eingesetzten Haltering 106 der in Richtung des Teils 101 mit einem vorspringenden umlaufenden Flanschring 107 versehen ist. In diesem Flanschring 107 ist eine Klemmscheibe 108 eingepreßt. Zwischen der Klemmscheibe 108 und dem Haltering 106 befindet sich eine Filtermembran 109. Diese Filtermembrane 109 ist über in der Klemmscheibe 108 angeordnete Stege 110 abgestützt. Die Stege 110 ragen über eine sich in Richtung des Halterings 106 erweiternde kegelförmige Öffnung vor, die in einen Auslaß 111 mündet, der seinerseits in den Drosselkanal 32 einmündet. Durch eine derartige Ausbildung wird verhindert, daß Schwebekörper oder kristallisierte Medikamententeile oder sonstige Verunreinigungen in den Drosselkanal 32 und von diesen in die Spritzennadel 5 kommen können, bzw. den Drosselkanal 32 verlegen. Dadurch wird bei selbsttätig wirkenden Injektionsvorrichtungen verhindert, daß eine in der Zeiteinheit abgegebenen Medikamentenmenge unvorhersehbar verringert wird und daß Nachteile für den Patienten entstehen können.

In Fig.15 ist gezeigt, daß der Drosselkanal 32 zwischen der Bohrung 33 und einer Auslaßbohrung 103 auch spiralförmig verlaufen kann.

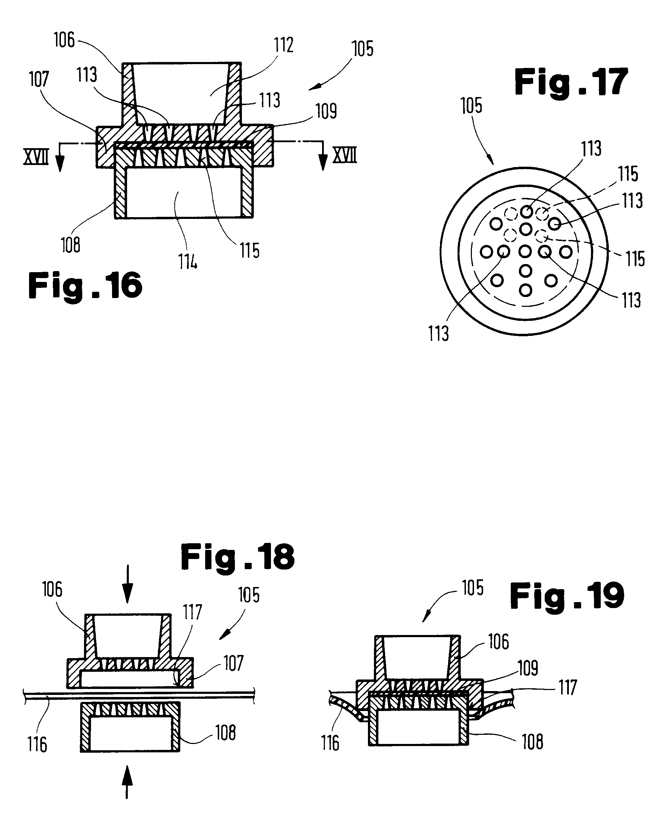

In Fig.16 und 17 ist ein Mikrofilter 105 in größerem Maßstab gezeigt. Der Mikrofilter 105 kann aus einem Haltering 106 mit einem umlaufenden Flanschring 107 gebildet sein. In einer Trennwand zwischen einer Einlaßöffnung 112 und der Filtermembran 109 sind in Richtung der Filtermembran 109 sich verjüngende beispielsweise kegelförmige Einlaßkanäle 113 angeordnet. In einer zwischen der Filtermembran 109 und einem Auslaß 114 angeordneten Trennwand sind Auslaßkanäle 115 vorgesehen, die sich ebenfalls in Richtung der Filtermembran 109 konisch verjüngen, beispielsweise kegelförmig ausgebildet sein können. Dadurch wird eine gleichmäßige Verteilung des über die Einlaßöffnung 112 einströmenden Medikaments über die gesamte Fläche der Filtermembran 109 erreicht, sodaß der Strömungswiderstand des Mikrofilters 105 relativ gering ist. Ein weiterer Vorteil der Einlaßkanäle 113 liegt darin, daß diese gleichzeitig als Grobpartikelfilter dienen können und Grobpartikel daher bereits vor der Filtermembran 109 ausgeschieden werden können.

Wie aus Fig.14 besser zu ersehen ist, können die Einlaßkanäle 113 gleichmäßig beispielsweise in konzentrischen Kreisen über die Trennwand zwischen der Einlaßöffnung 112 und Filtermembran 109 verteilt angeordnet sein. Bevorzugt sind die Auslaßkanäle 115 wie in Fig.17 durch strichlierte Linien angedeutet gegenüber den Einlaßkanälen 113 seitlich versetzt, sodaß das Medikament nicht nur senkrecht sondern auch in radialer Richtung die Filtermembran 109 hindurchtreten muß, wodurch die Filterwirkung eines derartigen Mikrofilters 105 zusätzlich erhöht werden kann.

In den Fig.18 und 19 ist gezeigt, wie eine derartige Filtermembran 109 die beispielsweise einen Durchmesser von 1 bis 2 mm aufweisen kann zwischen dem Haltering 106 und der Klemmscheibe 108 eingesetzt werden kann. Dazu werden der Haltering 106 und die Klemmscheibe 108 auf beiden Seiten eines Bandes 116 für die Filtermembran 109 angeordnet. Das Band 116 der Filtermembran 109 wird zwischen die beiden Teile eingebracht und danach wird - wie durch Pfeile in Fig.18 schematisch angedeutet die Klemmscheibe 108 von unten an das Band 116 angelegt, worauf der Haltering 106 von oben her auf die Klemmscheibe 108 aufgepreßt wird. Dadurch wirkt eine Innenkante 117 des Flanschrings 107 in Art eines Stanzmessers, stanzt aus dem Band 116 die Filtermembran 109 im gewünschten Durchmesser heraus und preßt sie gleichzeitig auf die unter dem Band 116 befindliche Klemmscheibe 108. Da dieses Zusammenpreßen des Halterings 106 mit der Klemmscheibe 108 und das Durchtrennen des Bandes 116 der Filtermembran 109 nahezu gleichzeitig erfolgt, wird die Filtermembran 109 in ihrer exakt positionierten und zugeschnittenen Lage innerhalb des Mikrofilters 105 positioniert. Damit kann das Zuführen derartiger kleiner Filtermembranen 109 und die Handhabung zum Einbau zwischen dem Haltering 106 und der Klemmscheibe 108 wegfallen. Der Mikrofilter 105 kann in der gezeigten Ausführungsform auch sehr einfach in größere Bauteile aus Kunststoff eingespritzt oder eingeschäumt werden, wodurch sich für einen derartigen Mikrofilter vielfältige Anwendungsmöglichkeiten vor allem in der Medizintechnik eröffnen.

Vor allem bei intravenös gespritzten Medikamenten ist dadurch die Gefahr von Thrombosen durch in die Blutbann eingeschleuste Fremdkörper wesentlich verringert.

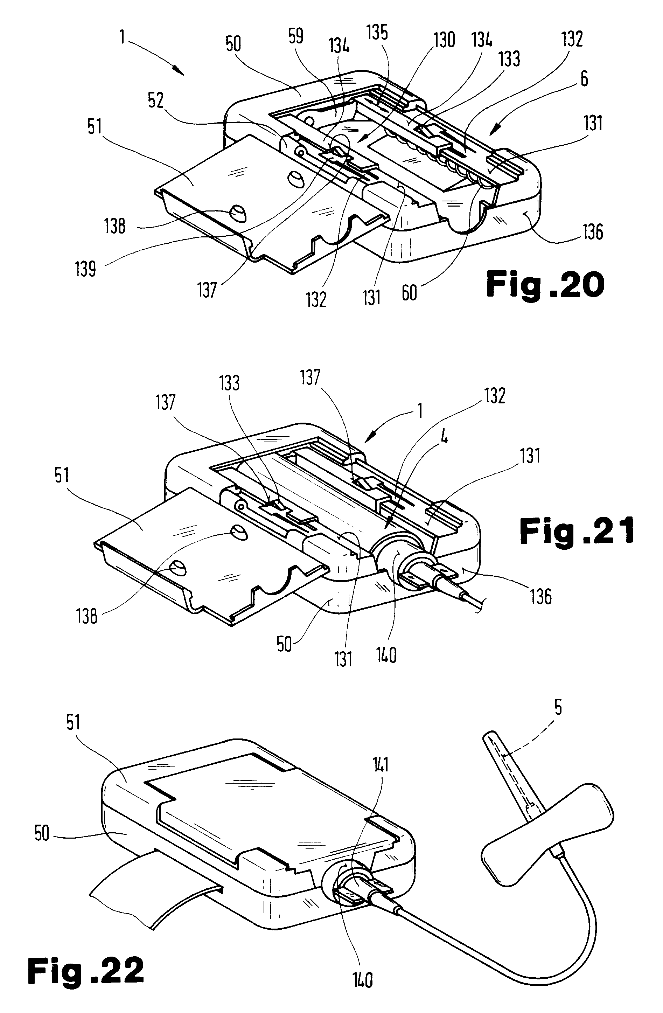

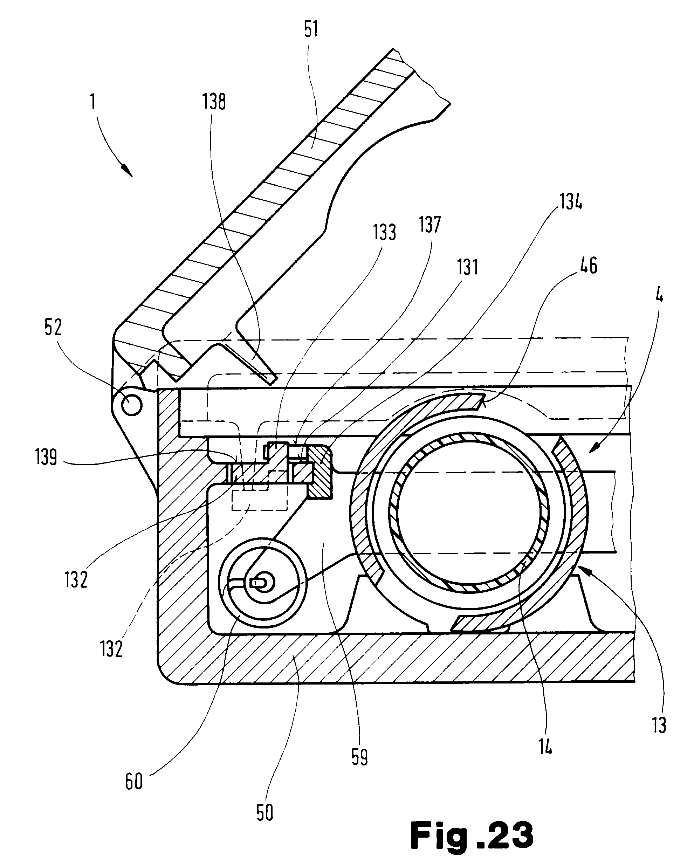

In den Fig.20 bis 23 ist eine andere Ausführungsform einer Injektionsvorrichtung 1 gezeigt. Da es sich dabei um eine Variante zu den vorhergehenden Ausbildungen handelt, wurden für gleiche Teile die gleichen Bezugszeichen verwendet. Die Injektionsvorrichtung 1 besteht aus einem wannenförmigen Gehäuseteil 50 und einem im Bereich einer Längsseitenkante des Gehäuseteiles 50 an einem Scharnier 52 gelagerten und einen Deckel bildenden Gehäuseteil 51. Im Gehäuseteil 50 ist die Antriebsvorrichtung 6, gebildet durch zwei beidseits der Ampulle 4 angeordnete auf Zug beanspruchte Federn 60, angeordnet. Diese sind beidseits eines Hohlraumes 130 für eine Ampulle 4 unter entlang von Seitenwänden des Gehäuseteiles 50 verlaufenden Gleitbahnen 131 angeordnet. Ein Teil jeder Gleitbahn 131 kann als federndes Schwenkelement 132 ausgebildet sein, auf dem ein die Oberfläche der Gleitbann 131 überragender Anschlag 133 angeordnet ist. Auf den Gleitbahnen 131 sind diese von der Seite der Ampulle 4 her U-förmig umfassende Führungselemente 134 längs eines Doppelpfeiles 135 verschiebbar gelagert. Die Führungselemente 134 sind an einem Stirnende durch den Querbalken 59 verbunden, der an den Enden der Federn 60 befestigt ist. Die anderen Enden der Federn 60 sind an einer dem Querbalken 59 entgegengesetzten Stirnwand 136 des Gehäuseteiles 50 verankert. Die Gegenanschläge 137 der Führungselemente 134 werden durch die bei gespannten Federn 60 wirkende Federkraft gegen die Anschläge 133 gepreßt und dadurch werden die Führungselemente 134 mit dem Querbalken 59 arretiert. Auf dem als Deckel ausgebildeten Gehäuseteil 51 sind an seiner im geschlossenen Zustand dem Gehäuseinneren zugewandten Oberfläche Druckstempel 138 angeordnet, die mit Anschlagflächen 139 der Schwenkelemente 132 zusammenwirken. Wird eine Ampulle 4 in den Hohlraum 130 zwischen Stirnwand 136 und Querbalken 59 eingelegt, so bleiben die Federn 60 gespannt bis der deckelartige Gehäuseteil 51 geschlossen wird. Beim Schließen des Gehäuseteiles 51 drücken die Druckstempel 138 die Schwenkelemente 132 aus der in Fig.23 in vollen Linien gezeigten Stellung in Richtung des Gehäuseteiles 50 und dies bewirkt, daß die auf den Schwenkelementen 132 angeordneten Anschläge 133 unter die Oberfläche der Gleitbahnen 131 in die in Fig.23 gezeigte strichlierte Stellung abgesenkt werden. Dadurch werden die die Gegenanschläge 137 bildenden Ausnehmungen der die Gleitbahnen 131 U-förmig umfassenden Führungselemente 134 freigegeben und das mit den Führungselementen 134 fest verbundene Querhaupt 59 übt durch die gespannten Federn 60 eine Druckkraft auf die Ampulle 4 oder den Ampullenkörper 14 in Richtung der Stirnwand 136 aus.

Wird dabei die Ampulle 4 in den Hohlraum 130 zwischen Stirnwand 136 und Querbalken 59 derart eingelegt, daß der Querbalken 59 mit dem Schlitz 46 deckungsgleich ist bzw. in diesen eingreift, so kommt es unmittelbar nach dem Schließen des Gehäuseteiles 51 und einer Freigabe der Führungselemente 134 durch das Hinunterdrücken zu einem Zusammendrücken des Balgs der Ampulle 4 und damit zu einem Auspressen des in der Ampulle 4 auf Vorrat gehaltenen Medikamentes.

Andererseits ist es aber auch möglich, die Ampulle 4 derart in den Hohlraum 130 einzusetzen, daß der Querbalken 59 auf der Stützvorrichtung 13 der Ampulle zur Anlage kommt. In diesem Fall wird auch nach dem Schließen des Gehäuseteiles 51, also des Deckels, trotz der Entriegelung der Anschläge 133 das Auspressen des Medikamentes aus der Ampulle 4 nicht eingeleitet, da eine Relativbewegung zwischen dem Querbalken 59 und der Stützvorrichtung 13 nicht möglich ist. Soll nun in diesem Fall eine Medikamentabgabe aus der Ampulle 4 erfolgen, so ist es erforderlich, die Stützvorrichtung 13 der Ampulle 4 soweit zu verdrehen, bis sich die Schlitze 46 mit dem Querhaupt 59 in einer deckungsgleichen Lage befinden. Dadurch kann das Querhaupt 59 auf den Ampullenkörper 14 einen Druck ausüben und diesen zusammenschieben. Dies bewirkt ein Auspressen des im Ampullenkörper 14 auf Vorrat gehaltenen Medikamentes. Das Verdrehen der Ampulle 4 kann dadurch erfolgen, daß ein aus dem Gehäuse 2 vorragender Teil der Ampulle 1, beispielsweise ein Verschlußnippel 140, der mit der Stützvorrichtung 13 bewegungsverbunden ist, solange verdreht wird bis das Querhaupt 59 in die Schlitze 46 einrastet. Die Drehbewegung der Ampulle 4 kann aber auch über einen Kupplungsteil 141 der mit dem Verschlußnippel 140 der Ampulle 4 über ein Gewinde oder einen Schnappverschluß bewegungsverbunden ist, in die das Auspressen des Medikamentes aus dem Ampullenkörper 14 ermöglichende Stellung verdreht werden. Der Kupplungsteil 141 ist über eine Schlauchleitung mit einer Spritzennadel 5 verbunden, über die das Medikament in die Blutbahn des Patienten injiziert werden kann.

Diese letztgenannte Ausführungsform, bei der das Auspressen des Medikamentes durch ein Verdrehen der Ampulle 4 aktiviert wird ist deshalb vorteilhaft, da beispielsweise das Einsetzen der Ampullen 4 in das Gehäuse 2 in Ruhe erfolgen kann, während bei einem plötzlichen Bedarf der Injektionsvorrichtung 1 zu deren Aktivierung lediglich eine kurze Drehbewegung erforderlich ist.

Selbstverständlich ist es anstelle des in den Fig.20 bis 22 beschriebenen Anschlages möglich, auch anders gestaltete bzw. in anderer Richtung schwenkbare Anschläge 133 zu verwenden. So ist es beispielsweise möglich, in der Gleitbahn 131 lediglich eine entsprechend große Öffnung vorzusehen, durch welche ein Anschlag 133 von unterhalb der Gleitbahn 133 in den Bereich der Führungselemente 134 vorragt. Durch eine entsprechende Gestaltung der Druckstempel 138 - außerhalb der Bewegungsbahn der U-förmigen Führungselemente 134 - kann dann beispielsweise der unabhängig von der Gleitbahn 131 am Gehäuseteil 50 gelagerte Anschlag 133 verschwenkt oder senkrecht zur Bodenfläche des Gehäuseteiles 50 verstellt werden. Bei entsprechender Gestaltung der Druckstempel ist es aber auch möglich, die einer oder beiden Gleitbahnen 131 zugeordneten Anschläge 133 beispielsweise um senkrecht zur Bodenfläche des Gehäuseteiles 50 verlaufende Achsen aus der Bewegungsbahn der Führungselemente 134 zu verschwenken.

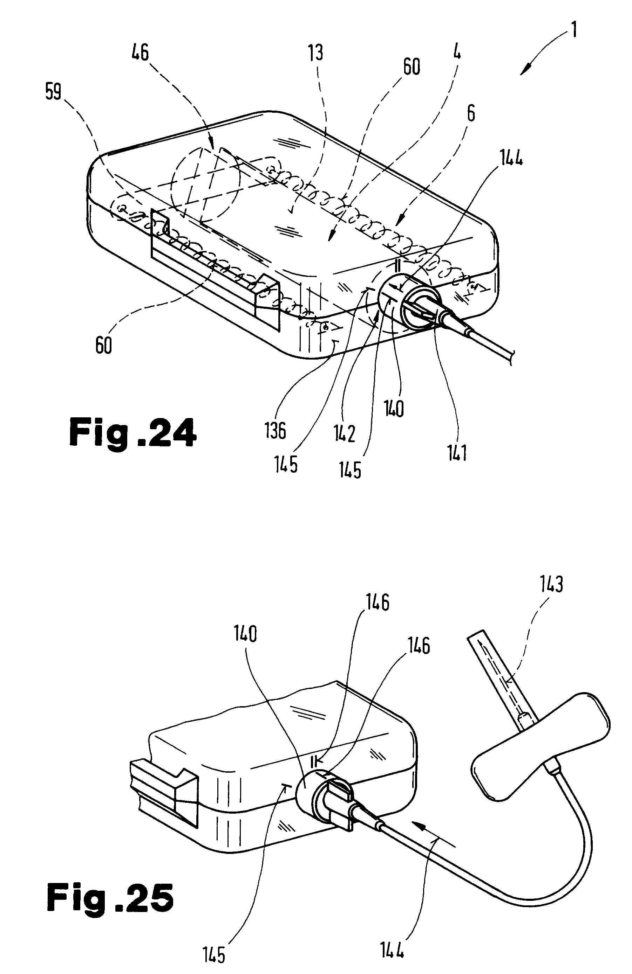

In den Fig.24 und 25 ist eine andere Ausbildung einer Injektionsvorrichtung 1 gezeigt. Dabei handelt es sich um ein Einweg-Abgabegerät. Innerhalb eines z.B. zweiteiligen versiegelten z.B. verschweißten Gehäuses ist die Ampulle 4 zwischen der Stirnwand 136 und dem Querhaupt 59 angeordnet. Die Führung und der Antrieb für das Querhaupt 59 entspricht dabei im wesentlichen der in den Fig.20 bis 23 beschriebenen Darstellung. Sie unterscheidet sich lediglich dadurch von der in den Fig.20 bis 23 beschriebenen Ausführungsform, daß die Anschläge 133 und die Gegenanschläge 137 sowie die Schwenkelemente 132 nicht vorgesehen sind. Die Arretierung der Ampulle 4 in ihrer Lage im Gehäuse 2 erfolgt durch die über die Federn 60 auf das Querhaupt 59 ausgeübte Zugkraft, die über die Stützvorrichtung 13 der Ampulle 4 diese gegen die gegenüberliegende Stirnwand 136 preßt und in dieser Lage fixiert. Die Ampulle 4 wird dabei so eingesetzt, daß die Schlitze 46 und das Querhaupt 59 gegeneinander verdreht und die Federn 60 der Antriebsvorrichtung 6 gespannt sind. Aus einer Öffnung 142 ragt in der Stirnwand 136 ein Verschlußnippel 140 für das Kupplungsstück 141 einer Kanäle 143. Der Verschlußnippel 140 bzw. das in diesen eingedrehte oder über einen Schnapp- oder Bajonettenverschluß mit diesem bewegungsverbundene Kupplungsstück 141 ist in Richtung eines Pfeiles 144 relativ zum Ampullenkörper 14 bzw. der Stützvorrichtung 13 in Achsrichtung der Ampulle 4 verstellbar. Bei dieser Verstellung bzw. Verschweißung des Verschlußnippels 140 in Richtung des Pfeiles 144 wird die Spurvorrichtung 22 der Verschluß- und Haltevorrichtung 15 der Ampulle 4 durchstoßen und dabei geöffnet. Wird im Anschluß an die in Achsrichtung der Ampulle 4 erfolgte Vorschubbewegung des Verschlußnippels 144, der gleichzeitig aber drehfest mit dem Stützkörper 13 gekuppelt ist, dieser um eine Längsachse der Ampulle verschwenkt, so kommt es nach einer ausreichenden Verdrehung der Stützvorrichtung 13 gegenüber dem Querbalken 59 zu einem Eingriff des Querbalken 59 in die Schlitze 46. Dies erfolgt dann, wenn der Stützkörper bzw. der Verschlußnippel 140 aus einer ersten Stellung 145 in eine zweite Stellung 146 gegenüber dem Gehäuse 2 verdreht ist. Befindet sich die Stützvorrichtung 13 in der Stellung 146 so greift der Querbalken 59 in die Schlitze 46 ein und wird durch die Federkraft in Richtung der Stirnwand 136 vorwärtsbewegt. Dadurch wird der Ampullenkörper 14 zusammengepreßt und das Medikament durch die Kanüle 143 ausgetragen.

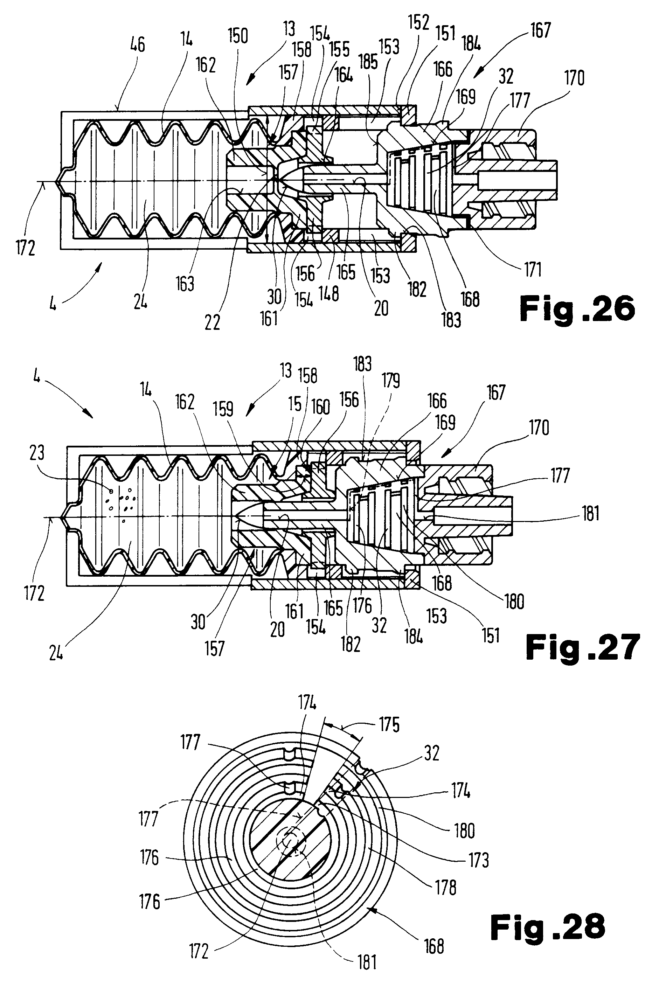

In den Fig.26 bis 29 ist eine andere Ausführungsvariante einer Ampulle 4 mit einer Drosselvorrichtung 167 gezeigt. Dabei werden für deren Beschreibung für gleiche Teile die gleichen Bezugszeichen verwendet.

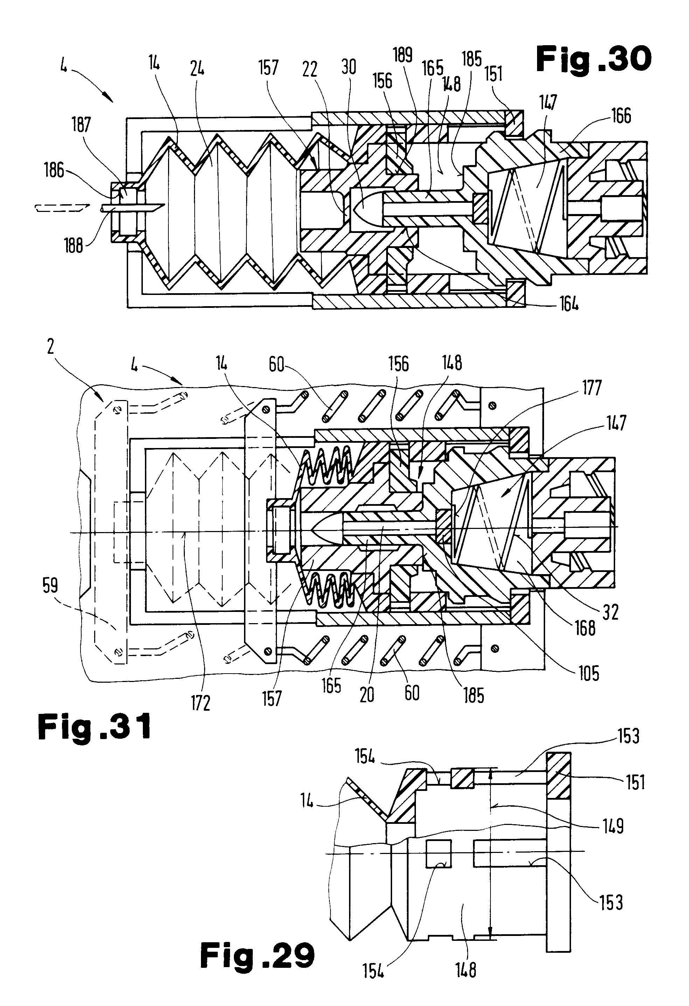

Der als Balg ausgebildete Ampullenkörper 14 der Ampulle 4 ist in der Stützvorrichtung 13 angeordnet. Der Ampullenkörper 14 weist zur Halterung in der Stützvorrichtung 13 einen zylinderförmigen Fortsatz 148 mit einem Außendurchmesser 149 - Fig.29 - auf. Der Außendurchmesser 149 entspricht im wesentlichen einem Innendurchmesser 150 der Stützvorrichtung 13 oder ist geringfügig größer als dieser Innendurchmesser 150, um einen exakten Sitz des Ampullenkörpers 14 in der Stützvorrichtung 13 zu ermöglichen. Zur Positionierung des Ampullenkörpers 14 ist der zylinderförmige Fortsatz - wie besser aus Fig.29 zu ersehen ist - mit einem Anschlagring 151 versehen, der an einer Stirnseite 152 der Stützvorrichtung 13 zur Anlage kommt. Wie weiters besser aus Fig.29 zu ersehen ist, ist der zylindrische Fortsatz 148 mit über den Umfang desselben verteilten Längsschlitzen 153 und von diesen in Längsrichtung distanzierten, ebenfalls über den Umfang verteilten Durchbrüchen 154, versehen. Die Durchbrüche 154 dienen dazu, um die mit den Durchbrüchen 154 in übereinstimmender Lage angeordneten Vorsprünge 155 einer Druckplatte 156 aufzunehmen. Mit dieser Druckplatte 156 wird ein die Verschluß- und Haltevorrichtung 15 bildender Verschlußpfropfen 157 in einem Halteflansch 158 des Ampullenkörpers 13 eingepreßt und dieser in der eingepreßten Position in dichter Anlage am Halteflansch 158 gehaltert in. Eine Distanz 159 - Fig.26 - zwischen dem Halteflansch 158 des Ampullenkörpers 13 und einer dieser zugewandten Stirnseite 160 der Druckplatte 156 ist kleiner als eine Dicke eines Stützringes 161 des Verschlußpfropfens 157 in entspanntem Zustand. Dadurch wird eine feste und dichte Anlage und ein Preßsitz des Verschlußpfropfens 157 im Halteflansch 158 des Ampullenkörpers 13 erzielt. Der Verschlußpfropfen 157 weist ferner einen in den Innenraum 24 des Ampullenkörpers 13 vorragenden Fortsatz 162 auf, der mit einer Bohrung 163 versehen ist, die gegenüber der Druckplatte 156 durch die Sperrvorrichtung 22, die z.B. durch eine dünne Haut des den Verschlußpfropfen 157 bildenden Kunststoffmateriales gebildet sein kann, verschlossen ist. In einer konzentrisch zur Bohrung 163 angeordneten Führungsbohrung 164 ist ein Ansatz 165 mit einer Spitze 30, die der Sperrvorrichtung 22 zugewandt ist, geführt. Der Ansatz 165 ist bevorzugt einstückig mit einem Aufnahmekörper 166 verbunden. Dieser Aufnahmekörper 166 lagert eine Drosselvorrichtung 167, die durch einen kegelstumpfförmigen Drosselkörper 168 gebildet ist. Dieser Drosselkörper 168 ist in eine Ausnehmung 169, die als kegelstumpfförmige Öffnung ausgebildet ist, eingesetzt. Der Drosselkörper 168 wird über einen in den Aufnahmekörper 166 eingesetzten Verschlußnippel 170, der durch eine Ultraschallverschweißung 171 mit dem Aufnahmekörper 166 bewegungsverbunden ist, in der gewünschten Position gehalten. Zum Drosseln des Durchganges des im Innenraum 24 des Ampullenkörpers 14 enthaltenen Medikamentes 23 ist am Umfang des Drosselkörpers 168 ein Drosselkanal 32 angeordnet.

Wie besser aus der Darstellung in Fig.28 ersichtlich ist, verläuft der Drosselkanal 32 konzentrisch zu einer Längsachse 172 des Ampullenkörpers 14 und wird durch auf der Manteloberfläche des Drosselkörpers 168 umlaufende Vertiefungen gebildet, deren Anfang 173 und Ende 174 um einen Winkel 175, der zwischen 10° und 45° vorzugsweise 20° beträgt, voneinander distanziert ist. Das Ende 174 eines Drosselkanalteiles 176 ist mit einem Anfang 173 eines in Richtung der Längsachse 172 distanzierten weiteren Drosselkanalteiles 176 über einen Verbindungskanal 177, der durch eine in Richtung einer mantelerzeugenden Geraden in der Kegelstumpfoberfläche verlaufenden Vertiefung gebildet ist, zu dem Drosselkanal 32 verbunden. Gleichermaßen wird das Ende 174 des weiteren Drosselkanalteiles 176 mit einem Anfang eines Drosselkanalteiles 178 verbunden. Wie aus den Darstellungen in den Fig.26 bis 28 weiters zu ersehen ist, ist eine durch die Bohrung 20 gebildete Zuleitung über einen radial verlaufenden Verbindungskanal 179 mit dem nächstliegenden Drosselkanalteil 176 verbunden. Ein Drosselkanalteil 180 ist über einen ebenfalls radial verlaufenden Verbindungskanal 177 mit einem Auslaßkanal 181, der zentrisch im Verschlußnipppel 170 angeordnet ist, verbunden.

Durch die Ausbildung des Drosselkanales 32 unter Verwendung von Vertiefungen in der Oberfläche des Drosselkörpers 158, wird aufgrund der konischen Oberfläche desselben in Verbindung mit dem gleichen konischen Verlauf der Ausnehmung 169, eine einwandfreie Abdichtung zwischen den einzelnen Drosselkanalteilen 176,178,180 sowie den entsprechenden Verbindungskanälen 177,179 erreicht. Diese Abdichtung reicht auch aus, um eine Abdichtung zwischen den Drosselkanalteilen bei einem hohen Druck im Medikament sicherzustellen.

Selbstverständlich wird der selbe Effekt auch erreicht, wenn die Vertiefungen für die Drosselkanalteile 176,178,180 bzw. die Verbindungskanäle 177,179 in der Ausnehmung 169 des Aufnahmekörpers 166 angeordnet sind.

Ein weiterer Vorteil der vorstehend beschriebenen erfindungsgemäßen Ausführungsform besteht darin, daß der Ampullenkörper 14 in die Stützvorrichtung 13 bis zur Anlage des Anschlagringes 151 an der Stirnseite 152 der Stützvorrichtung 13 eingeschoben werden kann, worauf der Innenraum 24 der Ampulle 4 mit einem Medikament 23 gefüllt werden kann. Ist eine ausreichende Menge an Medikament 23 eingebracht, so wird der Verschlußpfropfen 157 eingesetzt und mit der Druckplatte 156 so gegen den Halteflansch 158 des Ampullenkörpers 14 gepreßt, daß eine sterile Abdichtung und ein steriler Verschluß des Innenraumes 24 erreicht wird. Daran anschließend wird der Aufnahmekörper 166 unter elastischer Verformung des Anschlagringes 151 durch am Aufnahmekörper 166 angeordnete, den Längsschlitzen 153 zugeordnete Aufnahmenasen 182 so weit eingeschoben, bis der Anschlagring 151 in eine Vertiefung 183 am Aufnahmekörper 166 einrastet, wie dies in Fig.26 in vollen Linien gezeigt ist. Damit ist die Ampulle 4 soweit vorbereitet, daß Sie in die Halterung 3 des Gehäuses 2 der Injektionsvorrichtung 1 jederzeit eingesetzt werden kann. Soll nun die Entnahme des Medikamentes aus dem Innenraum 24 beginnen, so genügt es, den Aufnahmekörper 166 - wie in Fig.27 gezeigt - in die in vollen Linien gezeigte Stellung parallel zur Längsachse 172 und in Richtung des Verschlußpfropfens 157 so weit hineinzuschieben, bis Halterasten 184 nach elastischer Verformung des Anschlagringes 151 von Seite des Ampullenkörpers 14 am Anschlagring 151 anliegen. Während dieses Einschiebevorganges wird der Aufnahmekörper 166 mittels der Aufnahmenasen 182 geführt, sodaß Verklemmungen oder unerwünschte seitliche Versetzungen des Ansatzes 165 vermieden werden. Der Ansatz 165 wird dabei zusätzlich durch die Führungsbohrung 164 in der Druckplatte 156 geführt. Der Durchmesser des Ansatzes 165 entspricht dem Durchmesser der Bohrung 163 im Verschlußpfropfen 157 oder ist geringfügig größer, sodaß es nach dem Durchtrennen der Sperrvorrichtung 22 zu einem dichten Abschluß zwischen dem Ansatz 165 und dem Verschlußpfropfen 157 kommt. Zusätzlich ist durch die Wahl der Lage der Aufnahmenasen 182 der Vertiefung 183 und den Fixierrasten 184 sichergestellt, daß eine Stirnseite 185 des Aufnahmekörpers 166 dicht an den die Führungsbohrung 164 umgebenden Flächen der Druckplatte 156 anliegen. Damit ist mit großer Sicherheit ein Austritt von Medikamenten aus dem Innenraum 24 des Ampullenkörpers 14 während des Auspressens des Medikamentes verhindert.

In den Fig.30 und 31 ist eine weitere Ausführungsvariante einer Ampulle 4 dargestellt. Diese Ampulle 4 entspricht in wesentlichen Grundzügen der in den Fig.26 bis 29 beschriebenen Ausführungsform.

So ist der Ampullenkörper 14 mit dem an diesen anschließenden Fortsatz 148 entsprechend der Ausführungsform in Fig.29 ausgebildet. Um jedoch zu jedem beliebigen Zeitpunkt in den Innenraum 24 der Ampulle 4 jedes beliebige Medikament in der vom Arzt gewünschten Dosierung einbringen zu können, ist auf der vom Fortsatz 148 abgewandten Seite des Ampullenkörpers 14 eine Öffnung 186 angeordnet, die mit einer Scheibe 187 aus dauerelastischem Silikon dicht abgeschlossen ist. Diese Scheibe 187 aus dauerelastischem Silikon ermöglicht nach einem Durchstechen der Scheibe 187 mit einer Nadel 188 einer Injektionsspritze das Einbringen eines beliebigen Medikamentes in den Innenraum 24. Wird die Nadel 188 wieder in die mit strichlierten Linien gezeigte Stellung herausgezogen, so hat das dauerelastische Silikonmaterial aus dem die Scheibe 187 gebildet ist, die Eigenschaft, daß es die Durchstichöffnung dicht verschließt, sodaß während des nachfolgenden Auspressens des Medikamentes aus dem Innenraum 24 des Ampullenkörpers 14 dieses ausschließlich über die Drosselvorrichtung 47 ausströmen kann, wenn die Sperrvorrichtung 22 durch die Spitze 30 des Ansatzes 165 durchstoßen ist. Desweiteren ist bei dieser Ausführungsform sichergestellt, daß jener Bereich des Ansatzes 165, der die Sperrvorrichtung 22 durchstößt und in den Innenraum 24 der Ampulle eindringt, in jedem Fall steril gehalten wird. Dazu ist der Verschlußpfropfen 157 in Richtung des Anschlagringes 151 des Ampullenkörpers 14 verlängert und durchsetzt in einer Bohrung 189 die Druckplatte 156. Der Verschlußpfropfen weist in diesem Bereich die Führungsbohrung 164 für den Ansatz 165 auf. Dadurch, daß der Verschlußpfropfen 157 aus einem Dichtungsmaterial, beispielsweise einem Silikonkautschuk oder ähnlichem, besteht, ist ein dichter Abschluß zwischen dem Ansatz 165 und der Führungsbohrung 164 gegeben, sodaß keine Keime in den Bereich der Spitze 30 des Ansatzes 165 vordringen können. Desweiteren wird sichergestellt, daß nach dem Durchstoßen der Sperrvorrichtung 22 durch den Ansatz 165 die Stirnseite 185 des Aufnahmekörpers 166 dicht an den Verschlußpfropfen 157 anliegt, wodurch das Austreten von Medikament während des Auspressens desselben aus dem Innenraum 24 zuverlässig verhindert ist. Weiters unterscheidet sich die in den Fig.30 und 31 dargestellte Ausführungsform von der in den Fig.26 bis 28 beschriebenen dadurch, daß die Drosselvorrichtung 147 anstelle der konzentrisch um die Längsachse 172 umlaufenden Drosselkanalteile, einen schraubenlinienförmigen Drosselkanal 32 aufweist, der sich von einer Stirnseite des kegelstumpfartigen Drosselkörpers 168 zu dieser gegenüberliegenden Stirnseite erstreckt. Die Zu- und Abfuhr des Medikamentes zu bzw. vom Drosselkörper 147 erfolgt wie dies anhand der Fig.27 und 28 beschrieben wurde.

In Fig.31 ist weiters der Ampullenkörper 14 in unterschiedlichen Stellungen gezeigt. So zeigt die Darstellung in strichlierten Linien, die Lage des Querbalkens 59 und des Ampullenkörpers 14 nach dem Einlegen des Ampullenkörpers in das Gehäuse 2, bevor die Medikamentenabgabe beginnt. Wird die Bewegung des Querbalkens 59 entsprechend den im Zusammenhang mit den vorstehenden Ausführungsbeispielen beschriebenen Möglichkeiten freigegeben, so wird der Querbalken 59 durch die Federn 60 in Richtung der Drosselvorrichtung 147 gezogen und drückt dadurch den Ampullenkörper 14 zusammen, bis er die in vollen Linien dargestellte Endstellung, bei der das gewünschte Volumen an Medikament aus dem Ampullenkörper 14 über die Drosselvorrichtung 147 ausgetragen wurde, einnimmt. Der Ampullenkörper 14 ist dann ziehharmonikaartig zusammengeschoben, sodaß die einzelnen Bälge einander unmittelbar benachbart sind. Die in den noch verbleibenden Hohlräumen zwischen den Bälgen verbliebenen Medikamentenreste werden durch eine entsprechend höhere Dosierung der in den Ampullenkörper 14 eingebrachten Medikamentenmenge bereits mitkalkuliert, sodaß die gewünschte Medikamentenmenge in jedem Fall zur Ausgabe durch die Drosselvorrichtung 147 zur Verfügung steht.

Selbstverständlich ist es im Rahmen der vorliegenden Erfindung auch möglich, die auf den Ampullenkörper 14 auszuübende Druckkraft durch Druckfedern aufzubringen, die sich beispielsweise auf der dem Querbalken 59 zugewandten Stirnseite des Gehäuses abstützen können und über Haltearme mit dem Querbalken 59 gekuppelt sein können. Darüberhinaus ist es auch möglich, kraftbetätigte Antriebe, wie beispielsweise einen Hydraulikzylinder oder Miniaturelektromotoren mit Ritzel vorzusehen, die in mit dem Querbalken 59 verbundene Zahnstangen oder dgl. eingreifen. Es wäre auch denkbar, als kraftbetätigbaren Antrieb eine Spindel-Wandermutteranordnung zu verwenden, wobei in bevorzugter Weise dann die Wandermutter mit dem Querbalken 59 bewegungsverbunden wäre.

Wie in dieser Ausführungsform weiters gezeigt ist, ist zwischen dem Verbindungskanal 177 und der Bohrung 20 ein Mikrofilter 105 angeordnet, dessen Funktion und Aufbau bereits vorstehend beschrieben wurde.

Selbstverständlich ist es im Rahmen der vorliegenden Erfindung möglich jede beliebige Antriebsvorrichtung zum Entleeren der Ampulle 4 zu verwenden. Diese kann in der Injektionsvorrichtung oder auch außerhalb derselben angeordnet und über Leitungen mit dieser verbunden sein.

Darüberhinaus ist auch das Material aus welchem die Ampulle bzw. der Ampullenkörper hergestellt sein kann frei wählbar. Es ist jedoch darauf Bedacht zu nehmen, daß nur derartige Kunststoffe eingesetzt werden, die medikamentenverträglich sind, wie z.B. Silikone.

## Patentansprüche

1. Injektionsvorrichtung mit einem Gehäuse (2), in welchem eine Halterung (3) für eine verformbare, ein Medikament aufnehmende Ampulle (4) angeordnet ist, deren Körper (14) sackförmig oder durch einen Kunststoffaltenbalg ausgebildet ist und deren offenes Ende ihres einen Stirnendbereiches mit einer kombinierten Verschluß- und Haltevorrichtung (15) verschlossen ist, wobei diesem Stirnendbereich eine Spritzennadel (5) zugeordnet ist, die in einem Nadelträger (26,41,92) in der Verschluß- und Haltevorrichtung (15) gehaltert und mit einem Innenraum der Ampulle verbunden ist, wobei ferner eine Antriebsvorrichtung (6) vorgeschen ist, die einem der Spritzennadel zugewandten Stirnendbereich gegenüberliegenden weiteren Stirnendbereich der Ampulle zugeordnet ist, und die einen in Längsrichtung der Ampulle und in Richtung der Verschluß- und Haltevorrichtung verstellbaren Kolben (7,58) umfaßt, der mit Führungsteilen versehen ist, die in einer Führungsbahn (45) geführt sind, dadurch gekennzeichnet, daß die Ampulle (4) in einer sich zwischen ihren beiden Stirnendbereichen erstrechenden Stützvorrichtung (13) angeordnet ist, daß die Verschluß- und Haltevorrichtung (15) von einem stabilen Kunststoffkörper gebildet und in der Stützvorrichtung (13) lagefest gehaltert ist, daß die Stützvorrichtung (13) in der Halterung (3) des Gehäuses (2) gelagert ist, daß die Führungsbahn (45) in der Stützvorrichtung (13) im wesentlichen parallel zur Längsachse (10) der Ampulle (4) verläuft, und daß im Nadelträger (26,41,92) und/oder in der Verschluß- und Haltevorrichtung (15) und/oder in einem an diese anschließenden Zwischenstück (43) ein Drosselkanal (32) angeordnet ist, der den Innenraum (24) der Ampulle (4) mit der Spritzennadel (5) verbindet.

2. Injektionsvorrichtung nach Anspruch 1, dadurch gekennzeichnet, daß der Ampullenkörper (14) aus Kunststoff gebildet ist.

3. Injektionsvorrichtung nach einem der Ansprüche 1 oder 2, dadurch gekennzeichnet, daß die Verschluß- und Haltevorrichtung (15) einstückig mit der Ampulle (4) verbunden ist.

4. Injektionsvorrichtung nach einem der Ansprüche 1 bis 3, dadurch gekennzeichnet, daß die Verschluß- und Haltevorrichtung (15) mit der Ampulle (4) verschweißt ist.

5. Injektionsvorrichtung nach einem der Ansprüche 1 bis 4, dadurch gekennzeichnet, daß die Verschluß- und Haltevorrichtung (15) der Ampulle (4) in die Stützvorrichtung (13) eingeschweißt bzw. eingeklebt ist.

6. Injektionsvorrichtung nach einem der Ansprüche 1 bis 5, dadurch gekennzeichnet, daß die Verschluß- und Haltevorrichtung (15) aus zwei Teilen (17,18) besteht und vorzugsweise zwischen diesen eine Sperrvorrichtung (22), z.B. eine Membran angeordnet ist.

7. Injektionsvorrichtung nach einem der Ansprüche 1 bis 6, dadurch gekennzeichnet, daß die Verschluß- und Haltevorrichtung (15) eine Kupplungsvorrichtung (25) für einen Nadelträger (26) aufweist.

8. Injektionsvorrichtung nach einem der Ansprüche 1 bis 7, dadurch gekennzeichnet, daß die Kupplungsvorrichtung (25) durch einen in der Verschluß- und Haltevorrichtung (15) angeordneten Kupplungsteil (27) bestehend aus einer Bohrung mit Innengewinde und einen am Nadelträger (26) angeordneten Kupplungsteil (28) bestehend aus einem Außengewinde gebildet ist und diese vorzugsweise parallel zu einer Längsachse (10) der Ampulle (4) bzw. einer Auslaßöffnung der Ampulle (4) ausgerichtet sind.

9. Injektionsvorrichtung nach einem der Ansprüche 1 bis 8, dadurch gekennzeichnet, daß der Sperrvorrichtung (22) eine Öffnungsvorrichtung (29) zugeordnet ist, die relativ zu dieser verstellbar und im Nadelträger (26,41,92) und bzw. oder in der Verschluß- und Haltevorrichtung (15) und bzw. oder in einem zwischen diesen angeordneten Zwischenstück (43) angeordnet ist.

10. Injektionsvorrichtung nach einem der Ansprüche 1 bis 9, dadurch gekennzeichnet, daß die Stützvorrichtung (13) rohrförmig, insbesondere mit kreisrundem Querschnitt ausgebildet ist.

11. Injektionsvorrichtung nach einem der Ansprüche 1 bis 10, dadurch gekennzeichnet, daß die Ampulle (4) rohrförmig, insbesondere mit kreisrundem Querschnitt ausgebildet ist.

12. Injektionsvorrichtung nach einem der Ansprüche 1 bis 11, dadurch gekennzeichnet, daß ein Außendurchmesser (38) der Ampulle (4) im wesentlichen einem Innendurchmesser der Stützvorrichtung (13) bzw. eines die Innenwände derselben tangierenden Hüllkreise entspricht.

13. Injektionsvorrichtung nach einem der Ansprüche 1 bis 12, dadurch gekennzeichnet, daß sich die Führungsbahn (45) von dem von der Verschluß- und Haltevorrichtung (15) distanzierten Stirnendbereich der Ampulle (4) bis in einen Übergangsbereich zwischen der Ampulle (4) und der Verschluß- und Haltevorrichtung (15) erstreckt.

14. Injektionsvorrichtung nach einem der Ansprüche 1 bis 13, dadurch gekennzeichnet, daß die Führungsbahn (45) durch zwei Längsschlitze (46) gebildet ist, die vorzugsweise im Bereich der Schnittpunkte einer Diametralen (47) mit den einander gegenüberliegenden Wandbereichen der Stützvorrichtung (13) angeordnet sind.

15. Injektionsvorrichtung nach einem der Ansprüche 1 bis 14, dadurch gekennzeichnet, daß die Öffnungsvorrichtung (29) des Nadelträgers (26,41,92) in etwa parallel zur Spritzennadel (5) angeordnet ist.

16. Injektionsvorrichtung nach einem der Ansprüche 1 bis 15, dadurch gekennzeichnet, daß die Öffnungsvorrichtung (29) des Nadelträgers (26,41,92) in etwa senkrecht zur Spritzennadel (5) und parallel zur Längsachse (10) der Ampulle (4) angeordnet ist.

17. Injektionsvorrichtung nach einem der Ansprüche 1 bis 16, dadurch gekennzeichnet, daß die Spritzennadel (5) senkrecht zur Längsachse (10) der Ampulle (4) angeordnet ist.

18. Injektionsvorrichtung nach einem der Ansprüche 1 bis 17, dadurch gekennzeichnet, daß die Stützvorrichtung (13) und bzw. oder der Nadelträger (26,41,92) an einem Widerlager der Injektionsvorrichtung (1,81,95), insbesondere dem Gehäuse (2,49,96) anliegen und ein Hub des Kolbens (7,58) der Antriebsvorrichtung (6) zumindest der Länge der Führungsbahn (45) der Stützvorrichtung (13) entspricht.

19. Injektionsvorrichtung nach einem der Ansprüche 1 bis 18, dadurch gekennzeichnet, daß die Antriebsvorrichtung (6) durch beidseits der Stützvorrichtung (13) angeordnete Antriebe, z.B. Federn (60) wie Schraubenfedern, gebildet ist.

20. Injektionsvorrichtung nach einem der Ansprüche 1 bis 19, dadurch gekennzeichnet, daß der Antriebsvorrichtung (6,61) eine Arretiervorrichtung, z.B. ein Arretierbügel (62), zugeordnet ist, mit der der Kolben (7,58) in einer von der Stützvorrichtung (13) distanzierten Endstellung festlegbar ist.

21. Injektionsvorrichtung nach einem der Ansprüche 1 bis 20, dadurch gekennzeichnet, daß die Arretiervorrichtung für den der Antriebsvorrichtung (61) zugeordneten Querbalken (59) aus einem eine Oberfläche einer in Längsrichtung der Ampulle (4) angeordneten Gleitbahn (131) für die Führungselemente (134) überragenden klinkenartigen Anschlag (133) gebildet ist.

22. Injektionsvorrichtung nach einem der Ansprüche 1 bis 21, dadurch gekennzeichnet, daß der klinkenartige Anschlag (133) um eine zur Vorschubrichtung der Führungselemente (134) etwa senkrecht verlaufende Achse verschwenkbar angeordnet ist.

23. Injektionsvorrichtung nach einem der Ansprüche 1 bis 22, dadurch gekennzeichnet, daß der klinkenartige Anschlag (133) auf einem Schwenkelement (132) angeordnet ist, dem an der diesen zugewandten Oberfläche des einen Deckel bildenden Gehäuseteiles (51) ein Druckstempel (138) zugeordnet ist.

24. Injektionsvorrichtung nach einem der Ansprüche 1 bis 23, dadurch gekennzeichnet, daß das Gehäuse (2,49,96) der Injektionsvorrichtung (1,81,95) zweiteilig ausgebildet ist und in einer Bodenplatte (55) eine Öffnung (56) für die Spritzennadel (5) angeordnet ist.

25. Injektionsvorrichtung nach einem der Ansprüche 1 bis 24, dadurch gekennzeichnet, daß der Kolben der Antriebsvorrichtung eine den Fortsatz der Ampulle aufnehmende Öffnung aufweist.

26. Injektionsvorrichtung nach einem der Ansprüche 1 bis 25, dadurch gekennzeichnet, daß die Antriebsvorrichtung (6) durch insbesondere parallel zur Längsachse (10) der Ampulle (4) ausgerichtete Schraubenspindelantriebe gebildet ist und der Kolben (7,58) mit Wandermuttern bewegungsverbunden ist.

27. Injektionsvorrichtung nach einem der Ansprüche 1 bis 26, dadurch gekennzeichnet, daß die Antriebsvorrichtung (6) durch fluidbeaufschlagte Zylinderkolbenanordnung (85) gebildet ist.

28. Injektionsvorrichtung nach einem der Ansprüche 1 bis 27, dadurch gekennzeichnet, daß der Antriebsvorrichtung (6) ein Druckspeicher (86) und bzw. oder ein Verdichter und bzw. oder eine Vakuumpumpe zugeordnet ist.

29. Injektionsvorrichtung nach einem der Ansprüche 1 bis 28, dadurch gekennzeichnet, daß der Ampulle (4) eine Steuervorrichtung (75) zugeordnet ist, die mit der Antriebsvorrichtung (6) und bzw. oder der Ampulle (4) zugeordneten Endschaltern (73) und bzw. oder mit einem Summer (76) und bzw. oder einer Lampe und gegebenenfalls mit einem Mengenmeßgerät verbunden ist.

30. Injektionsvorrichtung nach einem der Ansprüche 1 bis 29, dadurch gekennzeichnet, daß der Steuervorrichtung (75) Meßfühler (100) zugeordnet sind, die im Gehäuse (2,49,96) der Injektionsvorrichtung (1,81,95) und bzw. oder in einem diesen zugeordneten Band (69) angeordnet und zur Erfassung von Meßdaten, z.B. Puls, Blutdruck, Temperatur oder dgl. eines Patienten, ausgebildet sind.

31. Injektionsvorrichtung nach einem der Ansprüche 1 bis 30, dadurch gekennzeichnet, daß dem Gehäuse (2,49,96) der Injektionsvorrichtung (1,81,95) ein Rüttelfühler zugeordnet ist, der mit der Steuervorrichtung (75) zusammengeschaltet ist und die Steuervorrichtung (75) ein Steuerglied umfaßt, welches in Abhängigkeit von den Rüttelbewegungen eine unterschiedliche Menge an Medikament der Spritzennadel (5) zuführt.

32. Injektionsvorrichtung nach einem der Ansprüche 1 bis 31, dadurch gekennzeichnet, daß der Nadelträger (41) und bzw. oder das Zwischenstück (43) aus zwei Teilen (101,102) besteht, die bevorzugt in einer zur Längsachse (10) senkrechten Ebene geteilt sind und daß der Drosselkanal als Vertiefung in einem der beiden Teile (101 oder 102) angeordnet ist und die offene Seite der Vertiefung durch den anderen Teil (102 oder 101) verschlossen ist.

33. Injektionsvorrichtung nach einem der Ansprüche 1 bis 32, dadurch gekennzeichnet, daß zwischen der Öffnungsvorrichtung (29) und der Spritzennadel (5) ein Mikrofilter (105) angeordnet ist, wobei vorzugsweise eine der Spritzennadel (5) näherliegende Stirnseite des Mikrofilters (105) den Drosselkanal (32) abdeckt.

34. Injektionsvorrichtung nach einem der Ansprüche 1 bis 33, dadurch gekennzeichnet, daß der in einem der Teile (101 oder 102) angeordnete Drosselkanal (32) aus mehreren konzentrisch zur Längsachse (10) der Ampulle (4) verlaufenden Kreisringkanalteilen mit unterschiedlichen Durchmessern besteht, die sich jeweils über einen Winkel von z.B. 270 Grad erstrecken und in radialer Richtung abwechselnd miteinander verbunden sind.

35. Injektionsvorrichtung nach einem der Ansprüche 1 bis 34, dadurch gekennzeichnet, daß der in einem der Teile (101 oder 102) angeordnete Drosselkanal (32) aus einem konzentrisch zur Längsachse (10) der Ampulle (4) angeordneten Spiralkanal besteht.

36. Injektionsvorrichtung nach einem der Ansprüche 1 bis 35, dadurch gekennzeichnet, daß eine Drosselvorrichtung (147,167) aus zumindest zwei zueinander, sowie in etwa im rechten Winkel zu einer Mittelachse am Umfang eines kegelstumpfförmigen, vorzugsweise aus Kunststoff bestehenden Drosselkörpers (168) angeordneten Vertiefungen gebildet ist, die in Richtung der Mittelachse voneinander distanziert sind und Drosselkanalteile (176,178,180) bilden, deren Anfang (173) und Ende (174) jeweils einen Winkel (175) von 10 bis 45 vorzugsweise 20 in Umfangsrichtung voneinander distanziert ist, wobei das Ende (174) des einen Drosselkanalteiles (176,178) mit dem Anfang (173) des weiteren Drosselkanalteiles (176,178,180) über einen Verbindungskanal (177) zu einem Drosselkanal (32) verbunden ist.

37. Injektionsvorrichtung nach einem der Ansprüche 1 bis 36, dadurch gekennzeichnet, daß der Verbindungskanal (177) durch eine in Richtung einer mantelerzeugenden Geraden in der Kegelstumpfoberfläche verlaufende Vertiefung gebildet ist.

38. Injektionsvorrichtung nach einem der Ansprüche 1 bis 37, dadurch gekennzeichnet, daß die Drosselvorrichtung (147,167) durch zumindest einen in etwa schraubenlinienförmig verlaufenden am Umfang eines kegelstumpfförmigen vorzugsweise aus Kunststoff bestehenden Aufnahmekörpers (166) angeordneten Drosselkanales (32) gebildet ist.

39. Injektionsvorrichtung nach einem der Ansprüche 1 bis 38, dadurch gekennzeichnet, daß zwischen dem Drosselkanal (32) bzw. den Drosselkanalteilen (176,178,180) und einem Anfangsbereich eines Auslaßkanales (181) und einem Endbereich einer Zuleitung ein radial verlaufender Verbindungskanalteil (179) angeordnet ist.

40. Injektionsvorrichtung nach einem der Ansprüche 1 bis 39, dadurch gekennzeichnet, daß die Drosselvorrichtung (147,167) in einer kegelstumpfförmig ausgebildeten Ausnehmung (169) eines im wesentlichen zylindrischen und mit einem Ansatz versehenen vorzugsweise aus Kunststoff bestehenden Aufnahmekörpers (166) angeordnet ist und ein größerer Durchmesser der Ausnehmung (169) einem Basisdurchmesser und ein kleinerer Durchmesser der Ausnehmung einem Deckflächendurchmesser des kegelstumpfförmigen Drosselkörpers (168) entspricht.

41. Injektionsvorrichtung nach einem der Ansprüche 1 bis 40, dadurch gekennzeichnet, daß am in etwa zylinderförmigen Umfang des Aufnahmekörpers (166) Rasteneinrichtungen in etwa diametral gegenüberliegend angeordnet sind und ein zylinderförmiger und mit einer Spitze (30) versehener Ansatz (165) mit einer diesem im Zentrum längs einer Mittelachse durchdringenden Bohrung (20) angeordnet ist und an dem dem Ansatz (165) entgegengesetzten Ende des Aufnahmekörpers (166) ein die kegelstumpfförmige Ausnehmung (169) des Aufnähmekörpers (166) begrenzender und den Drosselkörper (168) haltender zylinderförmiger Verschlußnippel (170) vorzugsweise aus Kunststoff angeordnet ist der mit dem Aufnahmekörper (166) fest verbunden vorzugsweise verklebt oder verschweißt ist.

42. Injektionsvorrichtung nach einem der Ansprüche 1 bis 41, dadurch gekennzeichnet, daß der Mikrofilter (105) aus einem Haltering (106) und einer Klemmscheibe (108) besteht zwischen welchen eine Filtermembran (109) angeordnet ist, wobei vorzugsweise ein Innendurchmesser eines Flanschrings (107) des Halterings (106) den gleichen Durchmesser aufweist wie ein Außendurchmesser der Klemmscheibe (108).

43. Injektionsvorrichtung nach einem der Ansprüche 1 bis 42, dadurch gekennzeichnet, daß die einander zugewandten inneren und äußeren Umfangsflächen der Klemmscheibe (108) und des Halterings (106) als Preßsitz ausgebildet ist.

44. Injektionsvorrichtung nach einem der Ansprüche 1 bis 43, dadurch gekennzeichnet, daß der Verschlußpfropfen (157) über eine Druckplatte (156) im Fortsatz (148) des Ampullenkörper (14) abgestützt ist.

45. Injektionsvorrichtung nach einem der Ansprüche 1 bis 44, dadurch gekennzeichnet, daß in der Druckplatte (156) eine Führungsbohrung (164) für den Ansatz (165) angeordnet ist.

46. Injektionsvorrichtung nach einem der Ansprüche 1 bis 45, dadurch gekennzeichnet, daß der Verschlußpfropfen (157) die Druckplatte (156) in einer Bohrung (189) durchsetzt und mit einer Führungsbohrung (164) für den Ansatz (165) versehen ist.

47. Injektionsvorrichtung nach einem der Ansprüche 1 bis 46, dadurch gekennzeichnet, daß im Ampullenkörper (14) auf der vom Fortsatz (148) abgewandten Seite eine mit einer Scheibe (187) aus dauerelastischem Silikon abgedichtete Öffnung (186) angeordnet ist.

## Claims

1. An injection device with a housing (2) accommodating a holding device (3) for a deformable ampoule (4) that takes in a medicament and whose main ampoule section (14) is sack-like or formed of a plastic fold-up housing in the form of a bellows, and its open end at one of its frontal regions is closed by a combined sealing and holding device (15), whereby a hypodermic needle (5) is allocated to this frontal region , said needle being secured in a needle holder (26, 41, 92) positioned in the combined sealing and holding device (15) and connected to a space inside the ampoule, being further provided with a driving mechanism (6), that is allocated to a front end region facing the hypodermic needle opposite a further front end region of the ampoule, and which includes a piston (7, 58) which is adjustable in the longitudinal direction of the ampoule and in the direction of the combined sealing and holding device, and which comprises guiding parts, that are guided in a guide track (45), characterized in that the ampoule (4) is positioned in a supporting device (13) extending between its two front end regions and that the combined sealing and holding device (15) composed of a solid plastic section is secured in a stable manner in the supporting device (13), that the supporting device (13) is accommodated in the holding device (3) of the housing (2), that the guide track (45) in the supporting device (13) is essentially oriented parallel to the longitudinal axis (10) of the ampoule (4), and in that in the needle holder (26, 41, 92) and/or the combined sealing and holding device (15) and/or in a spacer (43) adjacent to the latter, a choking channel (32) is arranged, which connects the space inside (24) of the ampoule (4) with the hypodermic needle (5).

2. An injection device according to claim 1, characterized in that the main ampoule section (14) is made of plastic.

3. An injection device according to claim 1 or 2, characterized in that the combined sealing and holding device (15) is connected in one piece with the ampoule (4).

4. An injection device according to any one of claims 1 to 3, characterized in that the combined sealing and holding device (15) is welded to the ampoule (4).

5. An injection device according to any one of claims 1 to 4, characterized in that the combined sealing and holding device (15) of the ampoule (4) is welded, respectively cemented into the supporting device (13).

6. An injection device according to any one of claims 1 to 5, characterized in that the combined sealing and holding device (15) consists of two parts (17, 18), preferably with a blocking device (22), for example a diaphragm, between them.

7. An injection device according to any one of claims 1 to 6, characterized in that the combined sealing and holding device (15) has a coupling device (25) for a needle holder (26).

8. An injection device according to any one of claims 1 to 7, characterized in that the coupling device (25) consists of one coupling component (27) placed in the combined sealing and holding device (15), comprising a bore with an inside thread and another coupling component (28) comprising an outside thread, and that it extends preferably parallel to the longitudinal axis (10) of the ampoule (4), respectively an outlet of the ampoule (4).

9. An injection device according to any one of claims 1 to 8, characterized in that the blocking device (22) has a perforator (29), that can be moved relativ to said blocking device and that it is arranged in the needle holder (26,41,92) and/or in the combined sealing and holding device (15) and/or in a spacer (43) placed between them.

10. An injection device according to any one of claims 1 to 9, characterized in that the supporting device (13) is tubular, and in particular has a round cross-section.

11. An injection device according to any one of claims 1 to 10, characterized in that the ampoule (4) is tubular, and in particular has a round cross-section.

12. An injection device according to any one of claims 1 to 11, characterized in that an outside diameter (38) of the ampoule (4) essentially equals the inside diameter of the supporting device (13), respectively an enveloping circle (84) tangent to the inner surface thereof.

13. An injection device according to any one of claims 1 to 12, characterized in that the guide track (45) extends from the front end region of the ampoule (4) that is farthest from the combined sealing and holding device (15) to the transition region between the ampoule (4) and the combined sealing and holding device (15).

14. An injection device according to any one of claims 1 to 13, characterized in that the guide track (45) consists of two longitudinal slots (46), preferably in the vicinity of the points of intersection of a diametrical (47) with mutually facing areas of the walls of the supporting device (13).

15. An injection device according to any one of claims 1 to 14, characterized in that the perforator (29) of the needle holder (26, 41, 92) more or less parallels the hypodermic needle (5).

16. An injection device according to any one of claims 1 to 15, characterized in that the perforator (29) of the needle holder (26, 41, 92) is more or less perpendicular to the hypodermic needle (5) and parallel to the longitudinal axis (10) of the ampoule (4).

17. An injection device according to any one of claims 1 to 16, characterized in that the hypodermic needle (5) is perpendicular to the longitudinal axis (10) of the ampoule (4).

18. An injection device according to any one of claims 1 to 17, characterized in that the supporting device (13) and/or the needle holder (26, 41, 92) rests against a counterbearing, especially the housing (2, 49, 96) of the injection device (1, 81, 95)and in that the stroke by the piston (7, 58) of the activating mechanism (6) is at least as long as the track (45) of the support (13).

19. An injection device according to any one of claims 1 to 18, characterized in that the driving mechanism (6) is composed of activators on both sides of the supporting device (13), springs (60) for example, such as compression springs for instance.

20. An injection device according to any one of claims 1 to 19, characterized in that the driving mechanism (6, 61) is provided with a fixing device, a positioning strap (62) for example, which arrests the motion of the piston in an end position remote from the supporting device (13).

21. An injection device according to any one of claims 1 to 20, characterized in that the fixing device for the transverse strut (59) of the driving mechanism (61) consists of a catch-like stop (133) that projects beyond the surface of a slide (131) for the guides (134) along the ampoule (4).

22. An injection device according to any one of claims 1 to 21, characterized in that the catch-like stop (133) pivots around an axis that extends more or less perpendicular to the direction the guides (134) advance along.

23. An injection device according to any one of claims 1 to 22, characterized in that the catch-like stop (133) is arranged on a pivoting structure (132) to which is associated a punch (138) on the surface facing a housing component (51) that constitutes a cap.

24. An injection device according to any one of claims 1 to 23, characterized in that the housing (2, 49, 96) of the injection device (1, 81, 95) consists of two halves and that there is an opening (56) for the hypodermic needle (5) in the base plate (55).

25. An injection device according to any one of claims 1 to 24, characterized in that the piston of the driving mechanism has an opening that accommodates the continuation of the ampoule.

26. An injection device according to any one of claims 1 to 25, characterized in that the driving mechanism (6) is a threaded-spindle drive mechanism, especially one that parallels the longitudinal axis (10) of the ampoule (4) and the piston (7, 58) is coupled to it with traveling nuts.

27. An injection device according to any one of claims 1 to 26, characterized in that the driving mechanism (6) consists of a fluid-charged piston-and-cylinder mechanism (85).

28. An injection device according to any one of claims 1 to 27, characterized in that the driving mechanism (6) is associated with a pressure reservoir (86) and/or a compressor and/or a vacuum pump.

29. An injection device according to any one of claims 1 to 28, characterized in that the ampoule (4) is associated with a control device (75), that is connected to the driving mechanism (6) and /or to limit switches (73) associated with the ampoule (4) and/or to a buzzer (76) and/or to a lamp and optionally to a flowmeter.

30. An injection device according to any one of claims 1 to 29, characterized in that the control device (75) is associated with sensors (100) accommodated in the housing (2, 49, 96) of the injection device (1, 81, 95) and/or in a strip (69) associated with it and which measures such data as a patient's pulse, blood pressure, temperature, etc.

31. An injection device according to any one of claims 1 to 30, characterized in that the housing (2, 49, 96) of the injection device (1,81, 95) is associated with a motion detector, that is connected to the control device (75) and that the control device (75) includes a control element which, in accordance with the motion, varies the amount of medicament supplied to the hypodermic needle (5).

32. An injection device according to any one of claims 1 to 31, characterized in that the needle holder (41) and/or the spacer (43) consists of two parts (101, 102), preferably separated along a plane perpendicular to the longitudial axis (10), and that the choking channel is a depression in one of the two parts (101, 102) and that the open side of the depression is covered up by the other part (101, 102).

33. An injection device according to any one of claims 1 to 32, characterized in that there is a microfilter (105) between the perforator (29) and the hypodermic needle (5) and in that the front side of the microfilter (105) that is closer to the hypodermic needle (5) preferably covers up the choking channel (32).

34. An injection device according to any one of claims 1 to 33, characterized in that the choking channel (32) in one of the parts (101, 102) consists of several annular-channel sections concentric with the longitudinal axis (10) of the ampoule (4) and differing in diameter, each extending at an angle of 270° for example and communicating alternately and radially.

35. An injection device according to any one of claims 1 to 34, characterized in that the choking channel (32) in one of the parts (101, 102) is a helical channel concentric with the longitudinal axis (10) of the ampoule (4).

36. An injection device according to any one of claims 1 to 35, characterized in that a choking mechanism (147, 167) consists of at least two depressions facing each other and positioned more or less at a right angle to the central axis at the circumference of a truncoconical choking structure (168), preferably plastic, separated along the central axis and constituting choking channel sections (176, 178, 180) with their beginning (173) and end (174) separated by an angle (175) of 10° to 45°, preferably 20° along the circumference, whereby the end (174) of one choking channel section (176, 178) is connected with the beginning (173) of the other choking channel section (176, 178, 180) by means of a communicating channel (177) to a choking channel (32).

37. An injection device according to any one of claims 1 to 36, characterized in that the communicating channel (177) is a depression that parallels a generatrix in the surface of the truncated cone.

38. An injection device according to any one of claims 1 to 37, characterized in that the choking mechanism (147, 167) consists of at least one choking channel (32) that extends more or less in a helix along the circumference of a truncoconical, preferably plastic accommodating structure (166).

39. An injection device according to any one of claims 1 to 38, characterized in that a radial communicating-channel section (179) is arranged between the choking channel (32), respectively the choking-channel sections (176, 178, 180) and the initial section of an outlet channel (181) and an end section of an intake.

40. An injection device according to any one of claims 1 to 39, characterized in that the choking mechanism (147, 167) is accommodated in a truncoconical recess (169) in an essentially cylindrical, preferably plastic accommodating structure (166) with an extension, and that the longer diameter of the recess (169) equals the diameter of the base and that the smaller diameter of the recess equals the diameter of the top of the truncoconical choking structure (168).

41. An injection device according to any one of claims 1 to 40, characterized in that snap-in structures are arranged more or less diametrically opposite around the more or less cylindrical circumference of the accommodating structure (166) and that there is a cylindrical extension (165) with a point (30) and a bore (20) that extends through the center of this extension along a central axis, and that there is a cylindrical sealing nipple (170), preferably plastic, on the end of the accommodation structure (166) opposite of the extension (165), demarcating the truncoconical recess (169) of the accommodating structure (166) and securing the choking structure (168), and fastened , preferably cemented or welded to the accommodating structure (166).

42. An injection device according to any one of claims 1 to 41, characterized in that the microfilter (105) consists of a securing ring (106) and a clamping disk (108) with a filter membrane (109) arranged between them, whereby the inside diameter of an annular flange (107) of the securing ring (106) has the same diameter as the outside diameter of the clamping disk (108).

43. An injection device according to any one of claims 1 to 42, characterized in that the mutually facing inner and outer circumferential surfaces of the clamping disk (108) and the securing ring (106) fit tightly together.

44. An injection device according to any one of claims 1 to 43, characterized in that the stopper (157) rests on a pressure-application plate (156) in the continuation (148) of the main section (14) of the ampoule.

45. An injection device according to any one of claims 1 to 44, characterized in that there is a bore (164) in the pressure-application plate (156) for aligning the extension (165).

46. An injection device according to any one of claims 1 to 45, characterized in that the stopper (157) extends through a bore (189) in the pressure-application plate (156) and has a bore (164) for aligning the extension (165).

47. An injection device according to any one of claims 1 to 46, characterized in that there is an opening (186) sealed off with a disk (187) of permanently resilient silicone in the side of main section (14) of the ampoule that faces away from the continuation (148).

## Revendications

1. Dispositif d'injection avec un corps (2) et une fixation (3) disposée dans celui-ci pour une ampoule (4) déformable destinée à recevoir un médicament, dont le corps d'ampoule (14) est sous la forme d'un sac ou d'un soufflet réalisé en matière synthétique, dont l'extrémité ouverte en l'une de ses extrémités côté frontal est fermée par un dispositif combiné d'obturateur et de maintien (15), à quoi est associé à ladite extrémité côté frontal une aiguille d'injection (5) qui est maintenue par un dispositif d'obturateur et de maintien (15) dans un porte-aiguille (26, 41, 92) et reliée à une chambre intérieure de l'ampoule, à quoi en outre un dispositif entraîneur (6) est prévu qui est associé à une autre extrémité côté frontal de l'ampoule, opposée à celle-ci orientée vers l'aiguille d'injection, et qui inclue un piston deplaçable (7,58) en direction longitudinale de l'ampoule et en direction du dispositif combiné d'obturateur et de maintien, et qui est muni de guides étant guidés dans une glissière de guidage (45), caractérisé en ce que l'ampoule (4) est disposée dans un dispositif d'appui (13) s'étendant entre ses deux extrémités côté frontal, le dispositif d'obturateur et de maintien (15) est formé d'un corps stable en matière synthétique et que celui-ci est maintenu de manière inamovible dans le dispositif d'appui (13), le dispositif d'appui (13) est logé dans la fixation (3) du corps (2), la glissière de guidage (45) dans le dispositif d'appui (13) est essentiellement en parallèle avec l'axe en direction longitudinale (10) de l'ampoule (4), et un canal d'étranglement (32) est disposé dans le porte-aiguille (26, 41, 92) et/ou le dispositif d'obturation et de maintien (15) et/ou la pièce intercalaire (43) voisine de ceux-ci, qui relie la chambre intérieure (24) de l'ampoule (4) à l'aiguille d'injection (5).

2. Dispositif d'injection selon la revendication 1 caractérisé en ce que le corps d'ampoule (14) est réalisé en une matière synthétique.

3. Dispositif d'injection selon la revendication 1 ou 2, caractérisé en ce que le dispositif d'obturation et de maintien (15) est relié à l'ampoule (4) tout en une seule pièce.

4. Dispositif d'injection selon une des revendications 1 à 3, caractérisé en ce que le dispositif d'obturation et de maintien (15) est soudé à l'ampoule (4).

5. Dispositif d'injection selon une des revendications 1 à 4, caractérisé en ce que le dispositif d'obturation et de maintien (15) de l'ampoule (4) est soudé ou collé sur le dispositif d'appui (13).

6. Dispositif d'injection selon une des revendications 1 à 5, caractérisé en ce que le dispositif d'obturation et de maintien (15) est constitué par deux parties (17, 18) et qu'entre celles-ci est disposé un dispositif d'arrêt (22), un diaphragme par exemple.

7. Dispositif d'injection selon une des revendications 1 à 6, caractérisé en ce que le dispositif d'obturation et de maintien (15) présente un dispositif d'accouplage (25) pour un porte-aiguille (26).

8. Dispositif d'injection selon une des revendication 1 à 7, caractérisé en ce que le dispositif d'accouplage (25) est formé d'un demi-accouplement (27) disposé dans le dispositif d'obturation et de maintien (15) et constitué par un creux avec taraudage, et d'un demi-accouplement (28) disposé sur le porte-aiguille (26) et constitué par un filetage mâle, et de préférence, ceux-ci sont alignés parallèlement à l'axe en direction longitudinale (10) de l'ampoule (4) ou d'une ouverture de sortie de l'ampoule (49).

9. Dispositif d'injection selon une des revendications 1 à 8, caractérisé en ce qu'un dispositif d'ouverture (29) est associé au dispositif d'arrêt (22), qui par rapport à l'arrêt est plus ou moins deplaçable et disposé dans le porte-aiguille (26, 41, 92) et/ou le dispositif d'obturation et de maintien (15) et/ou dans une pièce intercalaire (43)disposée entre ceux-ci.

10. Dispositif d'injection selon une des revendication 1 à 9, caractérisé en ce que le dispositif d'appui (13) est en forme tubulaire, en particulier avec une coupe en travers ronde.

11. Dispositif d'injection selon une des revendication 1 à 10, caractérisé en ce que l'ampoule (4) est en forme tubulaire, en particulier avec une coupe en travers ronde.

12. Dispositif d'injection selon une des revendications 1 à 11, caractérisé en ce que le diamètre extérieur (38) de l'ampoule (4) correspond essentiellement au diamètre du noyau du dispositif d'appui (13) et/ou à une gaine qui enveloppe de manière tangente les parois internes de celui-ci.

13. Dispositif selon une des revendications 1 à 12, caractérisé en ce que la glissière de guidage (45) s'étend depuis la partie d'extrémité côté frontal espacé par le dispositif d'obturation et de maintien (15) de l'ampoule (4) jusqu'au voisinage de transition entre l'ampoule (4) et le dispositif d'obturation et de maintien (15).

14. Dispositif d'injection selon une des revendications 1 à 13, caractérisé en ce que la glissière de guidage (45) est constituée par deux fentes orientées longitudinalement (46) qui, de préférence, sont disposés au voisinage des points d'intersection d'une ligne diamétrale (47) avec les zones des parois opposée l'une de l'autre du dispositif d'appui (13).

15. Dispositif d'injection selon une des revendications 1 à 14, caractérisé en ce que l'organe d'ouverture (29) du porte-aiguille (26, 41, 92) est disposé à peu près parallèlement à l'aiguille d'injection (5).

16. Dispositif d'injection selon une des revendications 1 à 15, caractérisé en ce que l'organe d'ouverture (29) du porte-aiguille (26, 41, 92) est disposé à peu près perpendiculairement à l'aiguille d'injection (5) et parallèlement à l'axe en direction longitudinale (10) de l'ampoule (4).

17. Dispositif d'injection selon une des revendications 1 à 16, caractérisé en ce que l'aiguille d'injection (5) est disposée perpendiculairement à l'axe en direction longitudinale (10) de l'ampoule (4).

18. Dispositif d'injection selon une des revendications 1 à 17, caractérisé en ce que, le dispositif d'appui (13) et/ou le porte-aiguille (26, 41, 92) sont en appui sur un aboutissement du dispositif d'appui (13), en particulier sur le corps (2, 49, 96) et en ce qu'une course de piston (7, 58) du dispositif entraîneur (6) correspond au moins à la longueur de la glissière de guidage (45) du dispositif d'appui (13).

19. Dispositif d'injection selon une des revendications 1 à 18, caractérisé en ce que le dispositif entraîneur (6) est formé par des entraîneurs, par exemple des ressorts tels que des ressorts cylindriques disposés à tous les deux côtés du dispositif d'appui (13).

20. Dispositif d'injection selon une des revendications 1 à 19, caractérisé en ce que le dispositif entraîneur (6, 61) est associé à un dispositif d'arrêt, par exemple à une traverse d'arrêt (62) par laquelle le piston (7, 58) peut être fixé dans une position finale espacé du dispositif d'appui (13).

21. Dispositif d'injection selon une des revendications 1 à 20, caractérisé en ce que le dispositif d'arrêt pour le renforcement diagonal (59) associé au dispositif entraîneur (61) est constitué par une butée à cliquet (133) dépassant en hauteur une surface d'une glissière (131) pour les éléments de guidage (134) disposée en direction longitudinale de l'ampoule (4).

22. Dispositif d'injection selon une des revendications 1 à 21, caractérisé en ce que la butée à cliquet (133) est disposée de manière pivotable autour d' un axe à peu près perpendiculairement dans la direction d'avance des éléments de guidage (134).

23. Dispositif d'injection selon une des revendications 1 à 22, caractérisé en ce que la butée à cliquet (133) est disposée sur un élément pivotant (132) auquel est associé un poinçon (138) sur la surface tournée vers celui-ci du pièce du corps de seringue (51) formant un capuchon.

24. Dispositif d'injection selon une des revendications 1 à 23, caractérisé en ce que le corps (2, 49, 96) du dispositif d'injection (1, 81, 95) est constitué par deux pièces et une ouverture (56) pour l'aiguille d'injection (5) disposée dans une plaque de base (55).

25. Dispositif d'injection selon une des revendications 1 à 24, caractérisé en ce que le piston du dispositif entraîneur présente une ouverture recevant le prolongement de l'ampoule.

26. Dispositif d'injection selon une des revendications 1 à 25, caractérisé en ce que le dispositif entraîneur (6) se compose d'entraînements de tiges filetées, en particulier orientées parallèlement à l'axe en direction longitudinale (10) de l'ampoule (4) et le piston (7, 58) est relié de façon mobil à l'aide d'écrous mobiles.

27. Dispositif d'injection selon une des revendications 1 à 26, caractérisé en ce que le dispositif entraîneur (6) est constitué par une disposition cylindre-piston (85) à commande hydraulique.

28. Dispositif d'injection selon une des revendications 1 à 27, caractérisé en ce que le dispositif entraîneur (6) est associé à un réservoir sous pression (86) et/ou à un compresseur et/ou à une pompe à vide.

29. Dispositif d'injection selon une des revendications 1 à 28, caractérisé en ce que l'ampoule (4) est associée à un dispositif de réglage (75) qui est relié au dispositif entraîneur (6) et/ou aux interrupteurs de fin de course (73) de l'ampoule (4) et/ou un oscillateur (76) et/ou une lampe et le cas échéant, à un appareil à mesurer le volume.

30. Dispositif d'injection selon une des revendications 1 à 29, caractérisé en ce que le dispositif de réglage (75) est associé aux capteurs (100) logés dans le corps (2, 49, 96) du dispositif d'injection (1, 81, 95) et/ou disposés sur une bande (69) associée à ceux-ci et qui sont développés de façon à saisir des données de mesure, par exemple le rythme cardiaque, la tension artérielle, la température d'un malade, etc.

31. Dispositif d'injection selon une des revendications 1 à 30, caractérisé en ce que le corps (2, 49, 96) du dispositif d'injection (1, 81, 95) est associé à un capteur secoueur, qui est interconnecté à un dispositif de réglage (75) et le dispositif de réglage (75) inclue un élément de réglage, ce qui indépendamment de secousses alimente l'aiguille d'injection (5) en un volume variable de médicaments.

32. Dispositif d'injection selon une des revendications 1 à 31, caractérisé en ce que le porte-aiguille (41) et/ou la pièce intercalaire (43) est composé de deux pièces (101, 102), de préférence divisées en une plane perpendiculairement à l'axe en direction longitudinale (10) et le canal d'étranglement est disposé en forme d'une dépression dans l'une de deux pièces (101 ou 102) et en ce que le côté ouvert de la dépression est fermé par l'autre pièce (102 ou 101).

33. Dispositif d'injection selon une des revendications 1 à 32, caractérisé en ce qu'un microfiltre (105) est disposé entre l'organe d'ouverture (29) et l'aiguille d'injection (5), de préférence le côté frontal du microfiltre (105) près de l'aiguille d'injection (5) couvrant le canal d'étranglement (32).

34. Dispositif d'injection selon une des revendications 1 à 33, caractérisé en ce que le canal d'étranglement (32) logé dans l'une des pièces ( 101 ou 102 ) est constitué par plusieurs secteurs de canaux d'un anneau de cercle dont chacun présente des diamètres différents s'étendant sur un angle de l'ordre de 270° par exemple, et qui sont interconnectés en alternance dans une direction radiale.

35. Dispositif d'injection selon une des revendications 1 à 34, caractérisé en ce que le canal d'étranglement (32) disposé dans l'une de deux pièces (101 ou 102) est constitué par un canal spiralé disposé concentriquement à l'axe en direction longitudinale (10) de l'ampoule (4).

36. Dispositif d'injection selon une des revendications 1 à 35, caractérisé en ce qu'un dispositif d'étranglement (147, 167) se compose au moins de deux dépressions, mutuellement en regard et à peu près formant un angle droite avec l'axe central sur la périphérie d'un corps d'étranglement (168) en forme tronconique, de préférence, en une matière synthétique, celles-ci espacées l'une de l'autre en direction de l'axe central et formant des pièces de canaux d'étranglement (176, 178, 180) dont le commencement (173) et la fin (174) sont espacés l'un de l'autre d'un angle (175) de l'ordre de 10° à 45°, de préférence 20° dans la direction périphérique, après quoi la fin (174) d'une pièce de canal d'étranglement (176, 178) est reliée au commencement (173) de la pièce de canal d'étranglement suivante par un canal de jonction (177) à un canal d'étranglement (32).

37. Dispositif d'injection selon une des revendications 1 à 36, caractérisé en ce que le canal de jonction (177) est constitué par une dépression en direction d' une ligne droite formant une enveloppe dans la surface du cône.

38. Dispositif d'injection selon une des revendications 1 à 37, caractérisé en ce que le dispositif d'étranglement (147, 167) est constitué au moins par un canal d'étranglement (32), s'étendant à peu près en spirale et disposé sur la périphérie de la structure de réception (166) en forme tronconique, de préférence réalisé en une matière synthétique.

39. Dispositif d'injection selon une des revendications 1 à 38, caractérisé en ce qu' entre le canal d'étranglement (32) respectivement les pièces de canal d'étranglement (176, 178, 180) et au voisinage du commencement d'un canal de sortie (181) et de la fin d'une conduite d'alimentation, une pièce de canal de jonction (179) s'étendant en direction radiale est disposée.

40. Dispositif d'injection selon une des revendications 1 à 39, caractérisé en ce que le dispositif d'étranglement (147, 167)est disposé dans un creux (169) tronconique d'une structure de réception (166) essentiellement cylindrique et pourvue d' un embout, réalisé de préférence en une matière synthétique, et un plus grand diamètre du creux (169) est égal à un diamètre de base et un plus petit diamètre du creux (169) est égal à un diamètre du recouvrement d'un corps d'étranglement (168) tronconique.

41. Dispositif d'injection selon une des revendications 1 à 40, caractérisé en ce que des arrangements à crans, à peu près opposés diamètralement, sont diposés sur la périphérie à peu près cylindrique de la structure de réception (166) et qu'un embout (165) cylindrique avec une pointe (30), percé centralement le long d'un axe central par un forage (20) est disposé et sur l'extrémité de la structure de recéption (166) opposée à l'embout (165) est disposé un nipple de fermeture (170) en forme cylindrique, de préférence en une matière synthétique, limitant le creux (169) tronconique de la structure de réception (166) et maintenant le corps d'étranglement (168), qui est joint à la structure de réception (166), de préférence par collage ou soudage.

42. Dispositif d'injection selon une des revendications 1 à 41, caractérisé en ce que le microfiltre (105) est constitué par un anneau de blocage (106) et une disque de serrage (108) entre lesquels est disposé un diaphragme filtrant (109) à quoi de préférence un diamètre du noyau d'un anneau de bride (107) de l'anneau de blocage (106) présente le même diamètre qu'un diamètre extérieur du disque de serrage (108).

43. Dispositif d'injection selon une des revendications 1 à 42, caractérisé en ce que les surfaces circonférentielles extérieures et celles de l'intérieur, mutuellement en regard, du disque de serrage (108) and de l'anneau de blocage (106) sont formés en ajustage serré.

44. Dispositif d'injection selon une des revendications 1 à 43, caractérisé en ce que le bouchon de fermeture (157) est en appui à l'aide d' une plaque d'appui (156) sur le prolongement (148) du corps d'ampoule (14).

45. Dispositif d'injection selon une des revendications 1 à 44, caractérisé en ce qu'un forage pilote (164) pour l'embout (165) est disposé dans la plaque d'appui (156).

46. Dispositif d'injection selon une des revendicatins 1 à 45, caractérisé en ce que le bouchon de fermeture (157) traverse la plaque d'appui (156) par un vide de forure (189) et qu'il est muni d'un forage pilote (164) pour l'embout (165).

47. Dispositif d'injection selon une des revendications 1 à 46, caractérisé en ce que dans le corps d'ampoule (14) est disposé une ouverture (186) sur le côté détourné du prolongement (148), bouchée par un disque (187) en silicone élastiquement durable.
